# EUROPEAN PATENT APPLICATION

(11) **EP 4 599 807 A1**
(43) Date of publication of application: **13.08.2025**
(21) Application number: 24156843.5
(22) Date of filing: 09.02.2024
(51) Int. Cl.: A61F 13/15, A61F 13/475, A61F 13/494

(54) **WASHABLE AND REUSABLE ABSORBENT UNDERGARMENT**

(71) Applicant: Essity Hygiene and Health Aktiebolag, 405 03 Göteborg (SE)
(72) Inventor: BLOMSTRÖM, Philip, 405 03 Göteborg (SE); KNÖS, Anna, 405 03 Göteborg (SE)
(74) Representative: Hoffmann Eitle

(57) **Abstract**

The present disclosure relates to a washable and reusable absorbent undergarment (1) comprising an absorbent assembly (10), comprising a wearer facing top layer (15), a moisture barrier (12) layer, and optionally one or more intermediate layers (11, 16), wherein the wearer facing top layer (15) defines a circumferential outer edge (20) of the absorbent assembly (10). The circumferential outer edge (20) comprises one or more sealed edge sections (21a, 21b), each sealed edge section (21a, 21b) providing a continuous sealing along the sealed edge section (21a, 21b) and between the wearer facing top layer (15) and the moisture barrier (12).

## Description

### TECHNICAL FIELD

The disclosure relates to a washable and reusable absorbent undergarment.

### BACKGROUND

An absorbent article, such as an absorbent undergarment, is worn for the purpose of absorbing body fluids such as urine and vaginal fluids. A washable and reusable absorbent undergarment comprises fabrics of woven or knitted materials. Conventionally such a washable and reusable absorbent undergarment comprises an integral absorbent assembly arranged towards the crotch region of the wearer when the undergarment is worn. After use the absorbent undergarment is washed or laundered before reuse.

Reusable absorbent undergarments may be adapted for different volumes of body fluids, from low to moderate or heavy body fluids.

For reusable absorbent undergarments, also those suitable for moderate to heavy flows of body fluids, there is a demand for the undergarment being comfortable to wear as well as providing a good fit and being aesthetically pleasing. As such, there is a desire to provide reusable absorbent undergarments suitable for different flows of body fluids, wherein the required capacity for absorbing body fluids is provided without unnecessary bulk and/or aesthetically disadvantageous effects such as unwanted folds and/or creases.

### SUMMARY

The object of the invention is to meet one or more of the above-mentioned needs, or to provide a useful alternative.

This object is met by a washable and reusable absorbent undergarment according to claim 1, and a washable and reusable absorbent assembly for an undergarment according to claim 27.

As such, there is provided a washable and reusable undergarment comprising one or more fabric panels forming a front side and a back side being joined such that the undergarment forms a waist opening and a pair of leg openings with a crotch region extending between the leg openings. A central longitudinal axis of the undergarment extends along the one or more fabric panels of the undergarment in a direction from the back side and towards the front side, and a transversal axis of the undergarment extends along the one or more fabric panels, perpendicular to the central longitudinal axis and dividing the undergarment into the front side and the back side.

In view of the above, the central longitudinal axis will be located centrally as seen over a total transversal width of the undergarment. The transversal axis is to divide the undergarment into the front side and the back side. The location of the transversal axis, i.e., the division between the front side and the back side, may be determined by laying the complete undergarment out with the back of the undergarment towards a supporting surface and the front of the undergarment towards the viewer. The waist edge of the front of the undergarment is to be superposed over the waist edge of the back of the undergarment. When the undergarment is laid flat in this manner, the front side of the undergarment is towards the viewer, the back side of the undergarment is towards the supporting surface, and the transversal axis extends between them, i.e., along a fold between the front side and the back side formed in the crotch region of the undergarment. As such, the transversal axis may be a central transversal axis and located centrally as seen over a total longitudinal length of the undergarment.

The undergarment further comprises an absorbent assembly being arranged in at least part of the crotch region and comprising a wearer facing top layer and a moisture barrier being superimposed along a height axis, perpendicular to the longitudinal axis and the transversal axis. Optionally, the absorbent assembly further comprises one or more intermediate layers being superimposed along the height axis between the top layer and the moisture barrier.

The absorbent assembly is configured to absorb fluid insult, such as urine or menstrual fluids that may contact an insult region located in the central crotch area of the undergarment. The absorbent assembly may be permanently attached to one or more of the fabric panels. For example, the absorbent assembly may be permanently attached to one or more of the fabric panels by one or more mechanical joints such as e.g., seams.

The wearer facing top layer is arranged so as to face the skin of the user when the undergarment is in use.

The moisture barrier prevents liquid from leaking through the absorbent assembly and may for example be in the form of a liquid-impermeable barrier layer. In other variants however, the moisture barrier may be a liquid-impermeable coating provided e.g., on a garment facing side of an intermediate layer, or optionally on a wearer facing side of a fabric panel underlying the one or more optional intermediate layers.

The optional intermediate layer or layers may for example comprise one or more absorbent layers and/or an auxiliary layer such as a wicking layer.

Further, the wearer facing top layer of the absorbent assembly defines a circumferential outer edge of the absorbent assembly. For example, at least the wearer facing top layer may extend in a plane spanned by the transversal axis and the longitudinal axis so as to coincide essentially with the circumferential outer edge of the absorbent assembly.

When the undergarment is worn, the majority of a fluid insult, such as urine or menstrual fluids will contact a central crotch area of the undergarment, i.e., an insult region. Thus, portions of the circumferential outer edge surrounding the central crotch area and the insult region of the absorbent assembly are comparatively prone to leakages and require effective sealing to prevent fluid leakage from the absorbent assembly via the circumferential outer edge.

However, for some portions of the circumferential outer edge, the risk of leakage occurring is less. For example, portions of the circumferential outer edge that are relatively distanced from the insult region of the undergarment may not be that prone to leakage since the fluid received at the insult region needs to be distributed over a relatively long distance in order to reach portions close to the circumferential outer edge. Accordingly, portions of the circumferential outer edge which are deemed prone to less leakage may be provided with a continuous sealing which is flat and pliable while still providing sufficient sealing preventing fluid leakage from the absorbent assembly via the circumferential outer edge.

As proposed herein, the circumferential outer edge comprises one or more sealed edge sections wherein a bottom surface of the wearer facing top layer is sealingly attached to a top surface of the moisture barrier, and a bottom surface of the moisture barrier or a bottom surface of the wearer facing top layer is directly attached to a top surface of at least one fabric panel. The sealed edge sections provide a continuous sealing along the sealed edge section and between the wearer facing top layer and the moisture barrier.

Thus, the sealed edge section provide a continuous sealing so as to hinder fluid leakage from the absorbent assembly via the sealed edge section.

When used herein, "directly attached" includes that two adjacent layers may be directly joined by means of a fluid seal, to form a fluid sealing joint.

The sealed edge sections may be selected to be circumferential outer edge portions where the absorbent assembly is less prone to leakage. This could for example be where portions of the circumferential outer edge are relatively distant to an insult region of the absorbent assembly. As such, the fluid from a fluid insult can be distributed in a relatively large volume of the absorbent assembly before reaching the circumferential outer edge, meaning that little or even no fluid will reach the circumferential outer edge when the absorbent article is in use. Also, the sealed edge sections may be selected taking the comfort and fit of the undergarment into account. Examples of such regions include regions of the absorbent assembly facing the front and /or the back of the undergarment.

Thus, in each sealed edge section, the fluid seal provides a continuous sealing as seen in one or more directions along the circumferential direction, and along the sealed edge section.

As such, each sealed edge section may be arranged so as to hinder fluid leakage from the absorbent assembly to the outside of the circumferential outer edge via the sealed edge portion.

As understood from the above, the arrangement of sealed edge sections of the circumferential outer edge is thus efficient to hinder fluid leakage from the absorbent assembly via the sealed edge sections. As such, the one or more sealed edge sections will be efficient to hinder leakage of fluid from portions of the circumferential outer edge located relatively distanced from the insult region of the absorbent assembly to the outside of the circumferential outer edge, via the sealed edge sections.

Thus, at least a portion of the circumferential outer edge may for example form a front sealed edge section of the absorbent assembly and face the front of the undergarment. As such, at least a portion the front outer edge of the absorbent assembly may comprise a sealed edge section forming a continuous sealing along the sealed edge section and between the wearer facing top layer and the moisture barrier, so as to hinder fluid leakage from the absorbent assembly to the outside of the circumferential outer edge via the sealed edge section. When at least a portion of the front edge of the absorbent assembly is provided with a sealed edge section, the front edge region may be able to adapt to the user's body shape and movements. Moreover, the shape of the circumferential outer edge of the absorbent assembly in the front edge region may be more freely selected since there is less need to consider leakage from the absorbent assembly.

At least a portion of the circumferential outer edge may form a rear sealed edge section of the absorbent assembly and face the back of the undergarment. As such, at least a portion the rear outer edge of the absorbent assembly may comprise a sealed edge section forming a continuous sealing along the sealed edge section and between the wearer facing top layer and the moisture barrier, so as to hinder fluid leakage from the absorbent assembly to the outside of the circumferential outer edge via the sealed edge sections. When at least a portion of the rear edge of the absorbent assembly is provided with a sealed edge section, the rear edge region of the absorbent assembly may be able to adapt to the user's body shape and movements. Moreover, the shape of the circumferential outer edge of the absorbent assembly in the rear edge region may be more freely selected since there is less need to consider leakage from the absorbent assembly.

In a further example of the undergarment disclosed herein, the absorbent assembly may comprise a front sealed edge section facing the front of the undergarment, and a back sealed edge section facing the back of the undergarment. Thus, the circumferential outer edge of the absorbent assembly may comprise a portion of the front edge comprising a front sealed edge section facing the front of the undergarment, and a portion of the rear edge comprising a rear sealed edge section facing the back of the undergarment as outlined in the above.

With the term "circumferential outer edge portion" or "portion of the circumferential outer edge" as used herein, it is to be understood a portion of the circumferential outer edge as extending along the circumferential direction of the circumferential outer edge.

A first fluid seal is arranged to extend between the bottom surface of the wearer facing top layer and the top surface of the moisture barrier of the one or more sealed edge sections. The one or more optional intermediate layers in the absorbent assembly are arranged so as to not extend all the way to the circumferential outer edge of the absorbent assembly (as seen in a transversal and/or lateral direction). This will enable the formation of a flatter and more pliable seal providing for a better adaptation to the body of a wearer.

With the term "first fluid seal" as used herein is consequently meant a fluid seal providing a continuous sealing along the sealed edge section between the wearer facing top layer and the moisture barrier. As such, the first fluid seal provides a continuous sealing in the plane spanned by the longitudinal axis and the transversal axis, and between the wearer facing top layer and the moisture barrier.

The first fluid seal may extend along the circumferential outer edge of the one or more sealed edge sections at a distance from the circumferential outer edge of less than 5 mm, such as less than 2 mm, such as less than 1 mm. For example, the distance between the first fluid seal and the circumferential outer edge, as measured along any direction in the plane spanned by the longitudinal axis and the transversal axis, may be at less than 5 mm, such as less than 2 mm, such as less than 1 mm from the circumferential outer edge.

For example, the first fluid seal(s) may be arranged so as to substantially coincide with the circumferential outer edge of the absorbent assembly.

For example, the fluid seal may be arranged so as to join a circumferential outer edge of the wearer facing top layer with a circumferential outer edge of the moisture barrier. For example, the first fluid seal may extend generally along the circumferential outer edges of the wearer facing top layer and the moisture barrier of the absorbent assembly.

Optionally, in the one or more sealed edge sections, the first fluid seal may be arranged to extend along the circumferential outer edge and at a distance from the circumferential outer edge.

For example, the first fluid seal may be arranged to extend along the circumferential outer edge and at a constant distance from the circumferential outer edge, i.e., the first fluid seal have a shape following the contour of the circumferential outer edge.

In another example, the first fluid seal may be arranged to extend at a varying distance from the circumferential outer edge. Thus, the shape of the first fluid seal may deviate from the contour of the circumferential outer edge.

For example, the distance may be less than 5 mm. For example, the distance may be less than 2 mm, such as less than 1 mm.

That the wearer facing top layer and the moisture barrier are directly joined implies that the absorbent assembly may be relatively thin at the location of the first fluid sealing joint, thus reducing stiffness and bulkiness of the fluid sealing joint, to the benefit of the undergarment more easily adapting to the wearer's body shape and movements.

A second fluid seal is arranged to extend between the bottom surface of the moisture barrier or the bottom surface of the wearer facing top layer and the top surface of the at least one fabric panel along the circumferential outer edge at a distance from the circumferential outer edge in the one or more sealed edge sections. The second fluid seal may be provided at a distance less than 5 mm, such as less than 2 mm, such as less than 1 mm from the circumferential outer edge.

In addition to providing a further fluid sealing joint along the one or more sealed edge sections, the second fluid seal provides attachment of the absorbent assembly to the fabric panel in at least the region of the circumferential outer edge comprising sealed edge sections.

As such, a washable and reusable undergarment may be provided which is suitable for absorbing a flow of body fluids whilst at the same time providing a satisfactory fit to the body of the user and having an outer appearance similar to a regular undergarment.

In one variant of sealed edge section disclosed herein, there is provided an undergarment with an absorbent assembly wherein a first fluid seal is arranged to extend between the bottom surface of the wearer facing top layer and the top surface of the moisture barrier of the one or more sealed edge sections as explained above. The first fluid seal extends along the circumferential outer edge of the one or more sealed edge sections at a distance from the circumferential outer edge of less than 5 mm, such as less than 2 mm, such as less than 1 mm from the circumferential outer edge.

At least in the sealed edge sections, the one or more optional intermediate layers in the absorbent assembly are arranged so as to not extend all the way to the circumferential outer edge of the absorbent assembly (as seen in a transversal and/or lateral direction).

The first fluid seal provides a continuous sealing along the sealed edge section between the wearer facing top layer and the moisture barrier. As such, the first fluid seal provides a continuous sealing in the plane spanned by the longitudinal axis and the transversal axis, and between the wearer facing top layer and the moisture barrier.

A second fluid seal is arranged to extend between the bottom surface of the moisture barrier and the top surface of the at least one fabric panel along the circumferential outer edge at a distance from the circumferential outer edge in the one or more sealed edge sections. The second fluid seal may be provided at a distance less than 5 mm, such as less than 2 mm, such as less than 1 mm from the circumferential outer edge.

For example, the second fluid seal may be arranged so as to substantially coincide with the circumferential outer edge of the absorbent assembly.

For example, the second fluid seal may be arranged so as to join a circumferential outer edge of the wearer facing top layer and a circumferential outer edge of the moisture barrier layer. For example, the second fluid seal may extend generally along the circumferential outer edge of the wearer facing top layer and along the circumferential outer edge of the the moisture barrier layer of the absorbent assembly.

Optionally, in the one or more sealed edge sections, the second fluid seal may be arranged to extend along the circumferential outer edges of the wearer facing top layer and the moisture barrier layer at a distance from the circumferential outer edge. For example, the second fluid seal may be arranged to extend along the circumferential outer edges and at a constant distance from the circumferential outer edges of the wearer facing top layer and the moisture barrier layer, i.e., the second fluid seals have a shape following the contour of the circumferential outer edges of the wearer facing top layer and the moisture barrier layer along the sealed edge section.

In addition to providing a further fluid sealing joint along the one or more sealed edge sections, the second fluid seal provides attachment of the absorbent assembly to the fabric panel in at least the region of the circumferential outer edge comprising sealed edge sections.

As such, a washable and reusable undergarment may be provided which is suitable for absorbing a flow of body fluids whilst at the same time providing a satisfactory fit to the body of the user and having an outer appearance similar to a regular undergarment.

In another example, the second fluid seal may be arranged to extend at a varying distance from the circumferential outer edge of the wearer facing top layer. Thus, the shape of the second fluid seal may deviate from the contour of the circumferential outer edge of the wearer facing top layer.

In a further variant of sealed edge sections disclosed herein, there is provided an undergarment with an absorbent assembly wherein a first fluid seal is arranged to extend between the bottom surface of the wearer facing top layer and the top surface of the moisture barrier of the one or more sealed edge sections as explained above. The first fluid seal extends along the circumferential outer edge of the one or more sealed edge sections at a distance from the circumferential outer edge of less than 5 mm, such as less than 2 mm, such as less than 1 mm from the circumferential outer edge.

The first fluid seal provides a continuous sealing along the sealed edge section between the wearer facing top layer and the moisture barrier. As such, the first fluid seal provides a continuous sealing in the plane spanned by the longitudinal axis and the transversal axis, and between the wearer facing top layer and the moisture barrier.

Also in this variant, at least in the sealed edge sections, the one or more optional intermediate layers in the absorbent assembly are arranged so as to not extend all the way to the circumferential outer edge of the absorbent assembly (as seen in a transversal and/or lateral direction). However, the wearer facing top layer may in this variant extend beyond the moisture barrier, and the bottom surface of the wearer facing top layer is directly attached to the top surface of the fabric panel. Thus, in the sealed edge section(s), the wearer facing top layer extends past the outer edge of the moisture barrier (as seen in a transversal and/or lateral direction) to enable attachment of its bottom surface to a top surface of the fabric panel outside an outer edge of the moisture barrier layer.

The wearer facing top layer may extend beyond the moisture barrier by a distance from 1 to 30 mm, wherein the distance between the first fluid seal 24a and the outer edge of the wearer facing top layer may be 1-20 mm, preferably 1-10 mm, more preferably 3- 10 mm.

The second fluid seal is arranged to extend between the bottom surface of the wearer facing top layer and the top surface of the at least one fabric panel along the circumferential outer edge at a distance from the circumferential outer edge in the one or more sealed edge sections. The second fluid seal extends along the circumferential outer edge of the one or more sealed edge sections at a distance from the circumferential outer edge of less than 5 mm, such as less than 2 mm, such as less than 1 mm from the circumferential outer edge.

As such, a washable and reusable undergarment may be provided which is suitable for absorbing a flow of body fluids whilst at the same time providing a satisfactory fit to the body of the user and having an outer appearance similar to a regular undergarment.

For example, the second fluid seal may be arranged so as to substantially coincide with the circumferential outer edge of the absorbent assembly, i.e. with the circumferential outer edge of the wearer facing top layer.

For example, the second fluid seal may be arranged so as to join a circumferential outer edge of the wearer facing top layer with the fabric panel. For example, the second fluid seal may extend generally along the circumferential outer edge of the wearer facing top layer of the absorbent assembly.

Optionally, in the one or more sealed edge sections, the second fluid seal may be arranged to extend along the circumferential outer edge of the wearer facing top layer and at a distance from the circumferential outer edge.

For example, the second fluid seal may be arranged to extend along the circumferential outer edge and at a constant distance from the circumferential outer edge of the wearer facing top layer, i.e., the second fluid seal have a shape following the contour of the circumferential outer edge of the wearer facing top layer along the sealed edge section.

In another example, the second fluid seal may be arranged to extend at a varying distance from the circumferential outer edge of the wearer facing top layer. Thus, the shape of the second fluid seal may deviate from the contour of the circumferential outer edge of the wearer facing top layer.

For example, the distance may be less than 5 mm. For example, the distance may be less than 2 mm, such as less than 1 mm from the circumferential outer edge of the wearer facing top layer.

In addition to providing a further fluid seal along the one or more sealed edge sections, the second fluid seal provides attachment of the absorbent assembly to the fabric panel in at least the regions of the circumferential outer edge comprising sealed edge sections.

The fluid seals may be accomplished in several different ways.

The first and/or second fluid seals may be fluid sealing joints accomplished by sealingly joining the layers of the absorbent assembly. For example, a fluid sealing joint may be a fluid sealing adhesive joint, such as a hot melt adhesive joint, or a fluid sealing welded joint, such as a heat welded joint or an ultrasonic welded joint.

The fluid sealing joint may be a fluid sealing adhesive tape such as a double-sided adhesive tape wherein the adhesive tape is a tape with adhesive on both sides such that it may adhere to both an upper layer and a lower layer.

In one example, the first fluid seal may be accomplished by means of sealingly joining the layers of the absorbent assembly together with a first fluid sealing joint. For example, the wearer facing top layer and the moisture barrier may be directly joined by a first fluid sealing joint such as a fluid sealing adhesive joint, such as a hot melt adhesive joint, or a fluid sealing welded joint, such as a heat welded joint or an ultrasonic welded joint thereby providing an adhesive connection of the wearer facing top layer to the moisture barrier.

For example, the bottom surface of the wearer facing top layer and the top surface of the moisture barrier may be directly joined by a fluid sealing double-sided adhesive tape.

The second fluid seal may be accomplished by means of sealingly joining the layers of the absorbent assembly together with a second fluid sealing joint. For example, the bottom surface of the moisture barrier or the bottom surface of the wearer facing top layer may be directly joined to the top surface of the fabric panel by a fluid sealing joint such as a fluid sealing adhesive joint, such as a hot melt adhesive joint, or a fluid sealing welded joint, such as a heat welded joint or an ultrasonic welded joint thereby providing an adhesive connection of the wearer facing top layer or the moisture barrier to the fabric panel.

For example, the bottom surface of the moisture barrier or the bottom surface of the wearer facing top layer may be directly joined to the top surface of the fabric panel by a fluid sealing double-sided adhesive tape.

For example, and in one variant of the undergarment the first fluid seal may be accomplished by means of sealingly joining the bottom surface of the wearer facing top layer and the top surface of the moisture barrier by a first fluid sealing joint, and the second fluid seal may be accomplished by directly joining the bottom surface of the moisture barrier to the top surface of the fabric panel by a second fluid sealing joint. The first and second fluid sealing joints may be a fluid sealing adhesive joint, such a hot melt adhesive joint, or a fluid sealing welded joint, such as a heat welded joint or an ultrasonic welded joint. For example, the first and second fluid sealing joints may be accomplished by means of fluid sealing double-sided adhesive tape.

For example, and in a further variant of the undergarment the first fluid seal may be accomplished by means of sealingly joining the bottom surface of the wearer facing top layer and the top surface of the moisture barrier by a first fluid sealing joint, and the second fluid seal may be accomplished by directly joining the bottom surface of the wearer facing top layer to the top surface of the fabric panel by a second fluid sealing joint. The first and second fluid sealing joints may be a fluid sealing adhesive joint, such a hot melt adhesive joint, or a fluid sealing welded joint, such as a heat welded joint or an ultrasonic welded joint. For example, the first and second fluid sealing joints may be accomplished by means of fluid sealing double-sided adhesive tape.

As understood from the above, the arrangement of sealed edge sections of the circumferential outer edge is thus efficient to hinder fluid leakage from the absorbent assembly via the sealed edge sections. As such, the one or more sealed edge sections will be efficient to hinder leakage of fluid from a fluid insult, typically occurring in a fluid insult region of the absorbent assembly located relatively distant to the circumferential outer edge, via the sealed edge sections.

When the undergarment is worn, the majority of a fluid insult, such as urine or menstrual fluids will contact a central crotch area of the undergarment, i.e., the insult region. It has been realized that due to the proximity of the leg openings to the insult region, in combination with the wearer's movements, these areas of the undergarment are more prone to leakage than other areas.

In one variant of the undergarment described herein, a pair of sealed assembly side portions may be located opposite to each other and on each side of the longitudinal axis (y), wherein optionally each sealed assembly side portion extends longitudinally along at least a part of the crotch region of the undergarment.

In each sealed assembly side portion, a top surface of the wearer facing top layer is sealingly attached to the moisture barrier or to the one or more fabric panels. By virtue of a sealing attachment reaching from a top surface of the wearer facing top layer to the moisture barrier or to the one or more fabric panels in the sealed assembly side portions, the risk of leakage from the absorbent assembly may be further reduced. This may be desired in particular in the crotch region of the undergarment. However, the sealed assembly side portions may risk becoming more stiff and bulky than the sealed edge sections as described in the above.

In each sealed assembly side portion at least a part of the circumferential outer edge of the absorbent assembly follows the contour of a respective leg opening of the undergarment.

Each sealed assembly side portion may comprise a fluid sealing member providing a continuous sealing between the top surface of the wearer facing top layer and the a bottom surface of the moisture barrier or a bottom surface of the one or more fabric panels in the sealed absorbent assembly side portion, so as to hinder fluid leakage from the absorbent assembly to the outside of the circumferential outer edge via the sealed assembly side portion.

Thus, the sealed assembly side portion may comprise a fluid sealing member being arranged so as to hinder fluid leakage from the absorbent assembly to the outside of the circumferential outer edge via the sealed assembly side portion. A fluid sealing member is in this context a fluid seal providing a continuous sealing along the sealed assembly side portion and between the wearer facing top layer and the moisture barrier. The fluid sealing member in the sealed assembly side portions may e.g., be provided by a sealing strip arranged to be folded from the top surface of the wearer facing top layer, over the circumferential outer edge of the wearer facing top layer and any intermediate layers, over the circumferential outer edge of the moisture barrier, and to the bottom surface of the moisture barrier. Alternatively, the sealing strip may be arranged to be folded from the top surface of the wearer facing top layer, over the circumferential outer edge of the wearer facing top layer and any intermediate layers, over the circumferential outer edge of the moisture barrier, and over a circumferential outer edge of the one or more fabric panels, to the bottom surface of the one or more fabric panels.

The fluid sealing member may for example be an elastic tape.

Further, each sealed assembly side portion may extend longitudinally along at least part of the crotch region of the undergarment.

As may be understood from the above, the sealed assembly side portion may per se have an extension along the transversal as well as along the longitudinal axis. That the sealed assembly side portion extends longitudinally along at least part of the crotch region of the undergarment implies that the sealed assembly side portion has at least an extension along the longitudinal axis in at least part of the crotch region.

Depending on the size and the style of the undergarment, the longitudinal length of the crotch region may vary considerably.

Optionally, the sealed assembly side portions may have a longitudinal extension over the entire crotch region of the undergarment. For example, the sealed assembly side portions may have a longitudinal extension extending beyond the crotch region of the undergarment towards the front and/or rear of the undergarment. This variant may be particularly useful for undergarment styles having a relatively short crotch region, i.e., low leg cut styles, such as boxer or shorts styles.

Optionally, the sealed assembly side portions may have a longitudinal extension over only a part of the crotch region of the undergarment. For example, the sealed assembly side portions may have a longitudinal extension over from 20 to 80 % of the crotch region, such as over from 25 to 75 %. This variant may be particularly useful for undergarment styles having a relatively long crotch region, i.e., high leg cut styles, such as string or Brazilian styles.

Optionally, in some variants, in each sealed assembly side portion, at least a part of the circumferential outer edge of the absorbent assembly follows the contour of a respective leg opening of the undergarment.

Optionally, in each sealed assembly side portion, at least part of the circumferential outer edge of the absorbent assembly essentially coincides with the contour of a respective leg opening of the undergarment.

For example, when the sealed assembly side portions have a longitudinal extension over a part of the crotch region of the undergarment as mentioned in the above, the circumferential outer edge of the absorbent assembly may essentially coincide with the contour of the respective leg opening in the entire part of the crotch region.

The sealed assembly side portions will thus extend over at least a portion of the longitudinal extension of the leg openings of the undergarment. Optionally, the sealed assembly side portions may extend over the entire longitudinal extension of the leg openings of the undergarment.

Optionally, each of the fluid seals in the sealed assembly side portions may extend continuously over a longitudinal side sealing length. In other words, the side sealing length is the extension of the sealed assembly side portions as seen along the longitudinal axis.

For example, the longitudinal side sealing length may be at least 5 cm, such as at least 7 cm. For example, the longitudinal side sealing length may be at least 10 cm.

For example, the longitudinal side sealing length may be less than 45 cm, such as less than 35 cm.

As such, the longitudinal side sealing length may for example be in the range of from 5 to 45 cm, such as from 10 to 35 cm.

As such, the longitudinal side sealing length may be selected so as to provide suitable protection against fluid leakage.

However, as mentioned in the above, the construction of the sealing used in the sealed assembly side portions may increase the stiffness of the absorbent assembly at the location of the sealing.

By combining one or more sealed assembly side portions with one or more sealed edge sections as described herein, the sealed edge sections having flatter and more pliable seals, it has been found to be possible to provide an undergarment with adequate hindering of fluid leakage in combination with a better fit. For example, unwanted folds or creases as seen from a front or back side of the undergarment when worn by a user may be reduced or avoided.

Thus, in addition to a pair of sealed assembly side portions located opposite to each other and on each side of the longitudinal axis y and extending longitudinally along at least a part of the crotch region, the circumferential outer edge advantageously comprises one or more portions provided with sealed edge sections.

Optionally, the circumferential outer edge forms a rear edge portion being the portion of the circumferential outer edge located longitudinally between the rearmost longitudinal end of the sealed assembly side portions and a rearmost end of the absorbent assembly.

Optionally, the rear edge portion comprises one or more sealed rear edge sections.

An absorbent assembly having a circumferential outer edge forming a rear edge portion extending longitudinally from the rearmost longitudinal end of the sealed assembly side portions towards the back portion of the undergarment enables variants with an extended back side of the absorbent assembly, as may be particularly suitable for undergarments intended for night-time use. By such a rear edge portion comprising one or more sealed rear edge sections, better fit and more versatility when it comes to selecting the shape of the rear edge portion may be achieved as outlined in the above.

Optionally, the entire rear edge portion constitutes a sealed edge section. As such, the entire rear edge portion is sealed against leakage from the absorbent assembly.

The wearer facing top layer and the moisture barrier layer may extend beyond the one or more optional intermediate layers in at least a portion of the rear edge portion, such as in the entire rear edge portion. As such, the rear edge portion may be relatively thinner and therefore able to provide a better fit to the user.

Advantageously, the wearer facing top layer and the moisture barrier may extend beyond the one or more optional intermediate layers in at least a portion of the rear edge portion, such as in the entire rear edge portion extending longitudinally along the rear portion of the absorbent assembly. In other words, the one or more optional intermediate layers may extend no further than the sealed assembly side portions in a direction towards the rear of the absorbent assembly. As such, the rear portion of the absorbent assembly may be relatively thinner and therefore able to provide a better fit to the user.

The rear edge portion may have a Rear Edge Length along the longitudinal axis.

The Rear Edge Length of the rear edge portion may for example be at least 2 mm, such as at least 4 mm. Optionally, the rear edge length of the rear edge portion may be extended, for example being at least 2 cm, such as at least 4 cm or at least 8 cm. The rear edge length of the rear edge portion may optionally be adapted such that the absorbent assembly extends over the entire longitudinal extension of the back side of the undergarment, i.e., all the way to the waist of the undergarment, such as to a waistband of the undergarment. In other options, the rear edge portion may be adapted such that the absorbent assembly extends over a part of the extension of the back side of the undergarment, such that there is a distance between the waist, e.g., a waistband, of the undergarment and the back end of the absorbent assembly. For example, the rear edge length may be less than 40 cm, such as less than 30 cm or less than 25 cm. Optionally, the rear edge length may be from 2 cm to 40 cm, such as from 4 cm to 30 cm.

Alternatively, or in addition, the circumferential outer edge may form a front edge portion being the portion of the circumferential edge located longitudinally between the frontmost longitudinal end of the sealed assembly side portions and a frontmost end of the absorbent assembly.

Optionally, the front edge portion comprises one or more sealed front edge sections.

An absorbent assembly having a circumferential edge forming a front edge portion extending longitudinally from the frontmost longitudinal end of the sealed assembly side portions towards the front portion of the undergarment enables variants with an extended front side of the absorbent assembly, as may be particularly suitable for undergarment intended for night-time use. By such a front edge portion comprising one or more sealed front edge sections, better fit and more versatility when it comes to selecting the shape of the front edge portion may be achieved as outlined in the above.

Optionally, the entire front edge portion constitutes a sealed edge section. As such, the entire front edge portion is sealed against leakage from the absorbent assembly.

Optionally, the wearer facing top layer and the moisture barrier layer may extend beyond the one or more optional intermediate layers in at least a portion of the front edge portion, such as in the entire front edge portion. As such, the front edge portion may be relatively thinner and therefore able to provide a better fit to the user.

Optionally, the wearer facing top layer and the moisture barrier may extend beyond the one or more optional intermediate layers in the entire part of the absorbent assembly extending longitudinally along the front edge portion. In other words, the one or more optional intermediate layers may extend no further than the sealed assembly side portions in a longitudinal direction towards the front of the absorbent assembly. As such, the front edge portion of the absorbent assembly may be relatively thinner and therefore able to provide a better fit to the user.

The front edge portion may have a Front Edge Length along the longitudinal axis.

The Front Edge Length of the front edge portion may for example be at least 2 mm, such as at least 4 mm. Optionally, the front edge length of the front edge portion may be extended, such as may be 2 cm, such as at least 4 cm or at least 10 cm. The front edge length of the front edge portion may optionally be adapted such that the absorbent assembly extends over the entire longitudinal extension of the front side of the undergarment, i.e., all the way to the waist of the undergarment, such as to a waistband of the undergarment. In other options, the front edge portion may be adapted such that the absorbent assembly extends over a part of the extension of the front side of the undergarment, such that there is a distance between the waist, e.g., a waistband, of the undergarment and the front end of the absorbent assembly. For example, the front edge length may less than 40 cm, such as less than 30 cm or less than 25 cm. Optionally, the front edge length may be from 2 cm to 40 cm, such as from 4 cm to 30 cm.

For example, in some variants the absorbent assembly may comprise sealed assembly side portions and a sealed rear edge section with a rear edge length being equal to or greater than the side sealing length. This may be particularly suitable for undergarments intended for night-time use. For example, in some variants the absorbent assembly may comprise sealed assembly side portions, a sealed rear edge section and a sealed front edge section, wherein the rear edge length and/or the front edge length is equal to or greater than the side sealing length.

Optionally, as understood from the above, the circumferential outer edge may consist of a sealed front edge section, a sealed rear edge section, and a pair of sealed assembly side portions, the sealed assembly side portions interconnecting the front edge portion and the rear edge portion.

Optionally, and providing for many alternative variants, such as several of those mentioned in the above, the circumferential outer edge may consist of a first sealed edge section, a second sealed edge section, and a pair of sealed assembly side portions, the sealed assembly side portions interconnecting the first sealed edge section and the second sealed edge section.

The shape of the absorbent assembly may be adapted for example to the design of the undergarment, and in view of the need for sufficient absorption and satisfactory fit.

The absorbent assembly may comprise a front edge directed towards the front of the undergarment, a rear edge directed towards the rear of the undergarment, and a pair of side edges, wherein each side edge is at least partly directed towards a respective leg opening and connects the front edge and the rear edge.

Each side edge may be at least partly arranged along the respective leg opening of the undergarment. With "along" is herein meant that the side edge follows the shape of the leg opening and is arranged adjacent or at the respective leg opening.

For example, each side edge may be at least partly arranged at the respective leg opening of the undergarment. This implies that the outer edge of the undergarment towards each leg opening at least partly comprises the side edge of the absorbent assembly.

For example, a minimum transversal assembly crotch width of the absorbent assembly, i.e., a minimum distance between the side edges of the absorbent assembly, may be equal to a minimum transversal undergarment crotch width of the intermediate region of the undergarment.

For example, each side edge of the absorbent assembly may be arranged at the respective leg opening of the undergarment in at least the front intermediate region.

For example, essentially the entire each side edge of the absorbent assembly may be arranged at the respective leg opening of the undergarment.

In other variants, each side edge may be at least partly arranged along the respective leg opening of the undergarment, and with a distance to the respective leg opening. For example, each side edge may in its entirety be arranged along a respecting leg opening, and with a distance to the leg opening.

In some variants, the side edges may comprise concave portions, as seen towards the central longitudinal axis y. For example, the side edges may be generally concave, thus allowing for the shape of the absorbent assembly being relatively wider towards the front and rear of the undergarment, and narrower at a crotch region of the undergarment, as mentioned in the above.

In some variants, the front edge may be convex from the transversal axis. As such, at the location of the central longitudinal axis y, the front edge may protrude towards the front of the undergarment beyond the side edges of the absorbent assembly. This may be beneficial for the fit of the undergarment to the body and contribute to the avoidance of unwanted creases in the fabric panels.

The rear edge of the absorbent assembly may be shaped so as to allow for suitable fit and comfort to a user.

As outlined in the above, the absorbent assembly comprises a wearer facing top layer, a moisture barrier, and optionally one or more intermediate layers being superimposed along a height axis, perpendicular to the central longitudinal axis and the transversal axis, between the wearer facing top layer and the moisture barrier. As such, the absorbent assembly comprises at least two layers, i.e., the wearer facing top layer and the moisture barrier. Such an absorbent assembly may be particularly suited for a light to moderate flow of body fluids.

Optionally, the absorbent assembly comprises a wearer facing top layer, a moisture barrier, and at least one intermediate layer.

Optionally, the absorbent assembly comprises a wearer facing top layer, a moisture barrier, and at least two intermediate layers.

Optionally, the absorbent assembly comprises a wearer facing top layer, a moisture barrier, and at least three intermediate layers. As such, the absorbent assembly comprises at least four layers plus the moisture barrier, which in some variants is formed by a moisture barrier layer. Accordingly, the capacity of the absorbent assembly to absorb relatively high flows of body fluid may be further improved, for example to provide an absorbent assembly suited for a heavy flow of body fluids.

For example, the absorbent assembly may comprise no more than nine layers.

As such, the absorbent assembly may comprise two, three, four, five, six, seven or eight intermediate layers.

A "layer" as used herein is a material layer. A layer could be made out of a laminate material.

The longitudinal and transversal extension (i.e. the outer shape) of the absorbent assembly per se is at least defined by the longitudinal and transversal extension of the wearer facing top layer.

In some variants, one or more optional intermediate layers may be arranged have a longitudinal and transversal extension not coinciding with the extension of the absorbent assembly.

In some variants, one or more of the optional intermediate layers are arranged so as to not extend all the way to the circumferential outer edge of the absorbent assembly.

For example, all of the optional intermediate layers of the absorbent assembly may not have a longitudinal and transversal extension essentially coinciding with the extension of the absorbent assembly. I.e., all of the layers of the absorbent assembly do not have the same longitudinal and transversal extension.

In other variants, the longitudinal and/or transversal extension of the one or more optional intermediate layers differ from the longitudinal and/or transversal extension of the absorbent assembly.

The absorbent assembly disclosed herein may be made from synthetic materials or fibers, natural materials or fibers or blends thereof. The top layer may for example have a basis weight of 80 - 450 gsm, or optionally 80 to 200 gsm.

Optionally, one or more of the optional intermediate layers is an absorbent layer. For example, when the absorbent assembly comprises at least three intermediate layers, at least two of the at least three intermediate layers may be absorbent layers.

The basis weight of the absorbent layer(s) may for example be in the range 150-450 gsm.

For example, the absorbent layer(s) may be of naturally derived fibers, synthetic fibers, or a blend or a mixture of naturally derived and/or synthetic fibers.

Optionally, one or more of the optional intermediate layers may be a wicking layer. For example, the wicking layer may be arranged immediately adjacent the wearer facing top layer. Optionally, the basis weight of the wicking layer is 180-250 gsm.

The absorbent assembly of the undergarment may be manufactured together with the undergarment as a whole and hence one or more joints joining together the absorbent assembly may also join the absorbent assembly to the one or more fabric panels of the undergarment.

Alternatively, the absorbent assembly of the undergarment may be manufactured as a separate part. As mentioned in the above, the absorbent assembly may, also when manufactured as a separate item, be permanently attached to the undergarment using one or more joints.

Although the option that the absorbent assembly is permanently attached to the undergarment is preferred, the absorbent assembly could instead be releasably attached to the undergarment. For example, the garment-facing side of the absorbent assembly may comprise a fastening member intended for releasably fastening the absorbent assembly to an undergarment. Such a fastening member may be adapted for direct attachment to a fabric panel of an undergarment, or the fastening member may be adapted for attachment to a corresponding fastening member of the undergarment.

Hence, in a second aspect, there is provided a washable and reusable absorbent assembly for an undergarment, the assembly comprising a wearer facing top layer, a moisture barrier, and optionally one or more intermediate layers being superimposed along a height axis, perpendicular to the longitudinal axis and the transversal axis, between the wearer facing top layer and the moisture barrier, the wearer facing top layer defining a circumferential outer edge of the absorbent assembly.

At least a portion of the circumferential outer edge of the absorbent assembly comprises one or more sealed edge sections wherein a bottom surface of the wearer facing top layer is sealingly attached to a top surface of the moisture barrier, and a bottom surface of the moisture barrier or the wearer facing top layer is configured to be directly attached to a top surface of at least one fabric panel of the undergarment, each sealed edge section providing a continuous sealing along the sealed edge section between the wearer facing top layer and the moisture barrier so as to hinder fluid leakage from the absorbent assembly to the outside of the circumferential outer edge via the sealed edge section.

As such, all features and advantages explained in the above relating to the absorbent assembly of an undergarment may be applied to the absorbent assembly per se and are encompassed by the present disclosure.

### BRIEF DESCRIPTION OF THE DRAWINGS

The disclosure will be described in greater detail below with reference to the figures shown in the appended drawings, wherein
- Fig. 1: shows a front view of a variant of an absorbent undergarment comprising an absorbent assembly;
- Fig. 2a: shows a view from the wearer-facing side of a first version of the undergarment of Fig. 1 when in a flat laid-out state with the joints between the front side and the back side of the undergarment being removed;
- Fig. 2b: shows a view from the wearer-facing side of a second version of the undergarment of Fig. 1 when in a flat laid-out state with the joints between the front side and the back side of the undergarment being removed;
- Fig. 2c: shows a view from the wearer-facing side of a third version of the undergarment of Fig. 1 when in a flat laid-out state with the joints between the front side and the back side of the undergarment being removed;
- Fig. 3a: shows a front view of another variant of an absorbent undergarment comprising an absorbent assembly;
- Fig. 3b: shows a view from the wearer-facing side of a first version of the undergarment of Fig. 3 when in a flat laid-out state with the joints between the front side and the back side of the undergarment being removed;
- Fig. 3c: shows a view from the wearer-facing side of a second version of the undergarment of Fig. 1 when in a flat laid-out state with the joints between the front side and the back side of the undergarment being removed;
- Fig. 4: is a schematic cross-sectional view of a variant of a sealed assembly side portion through an absorbent assembly, such as may be used for example in the undergarments of Fig 1 to 3c, and
- Figs. 5a-c: are schematic cross-sectional views of different variants of sealed edge section joints through an absorbent assembly, such as may be used for example in the undergarments of Figs. 1 to 3c.

### DETAILED DESCRIPTION

Different aspects of the present disclosure will be described more fully hereinafter with reference to the enclosed drawings. The embodiments of the absorbent undergarment disclosed herein can, however, be realized in many different forms, such as different sizes and absorption levels, and should not be construed as being limited to the aspects set forth herein. In all figures in the following detailed description, the same reference numerals will be used to indicate the same elements.

As used herein, the term "absorbent undergarment" refers to garments that are worn and intended to be placed against the skin of the wearer to absorb and contain body fluids such as urine and vaginal fluids including menstrual fluid. The undergarment may for example be an underwear, underpants, a panty, or a swimwear of a fabric. The undergarment is intended for adult users. The undergarment may be intended for female use. The undergarment according to the present disclosure is washable, i.e., intended to be laundered or otherwise restored after use for reuse as a sanitary article.

By "washable" it is meant that the absorbent undergarment may be cleaned by laundering. The absorbent undergarment may thus be subjected to an aqueous solution containing detergent without losing its structural features. This aqueous solution may be heated as part of the laundering process, e.g., to 40°C or 60°C.

The term "fabric" as used in the present disclosure may refer to single or multiple layers of fabrics. The fabric may be knitted or woven.

By "permanently attached", it is meant that the absorbent assembly is not intended to be separated from the body fabric before, during, or after use. The absorbent assembly is not necessarily directly bonded to the fabric panels, but instead may be attached to the fabric via auxiliary parts such as leg opening edgings or reinforcement patches.

Knitting is a method of constructing a fabric by interlocking a series of loops of one or more yarns. There are two major classes of knitting namely warp and weft knitting.

Weaving is a method or process of interlacing two yarns of similar materials so that they cross each other at right angles to produce woven fabric. The warp yarns run lengthwise in the fabric and the filling threads (weft) or picks, run from side to side.

Jacquard is a system of weaving or knitting that utilizes a versatile pattern mechanism to produce intricate designs by using punch cards controlling the patterns produced. Jacquard knit may be valid for circular, flatbed, warp or weft knit.

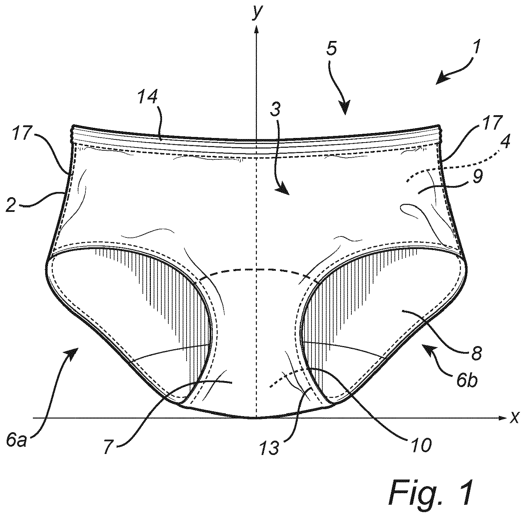

Fig. 1 illustrates as an example of a variant of a washable and reusable absorbent undergarment 1 in the form of a panty. As outlined in the above, the undergarment 1 as proposed herein may however have various styles, such as briefs, boxer, hipster, high waist, string, Brazilian, or other suitable undergarment styles. The undergarment may be in a form intended to be worn underneath other clothing. Alternatively, the undergarment in the form of a washable absorbing clothing such as pajamas pants or shorts, yoga pants, swimwear, sports pants or shorts.

The undergarment 1 may comprise one or more fabric panels 2 forming a front side 3 and a back side 4 being joined such that the undergarment 1 forms a waist opening 5 and a pair of leg openings 6a, 6b with a crotch region 7 extending between the leg openings 6a, 6b. A central longitudinal axis y of the undergarment 1 extends along the one or more fabric panels 2 of the undergarment 1 in a direction from the back side 4 and towards the front side 3, and a transversal axis x of the undergarment 1 extends along the one or more fabric panels 2, perpendicular to the central longitudinal axis y and dividing the undergarment 1 into the front side 3 and the back side 4.

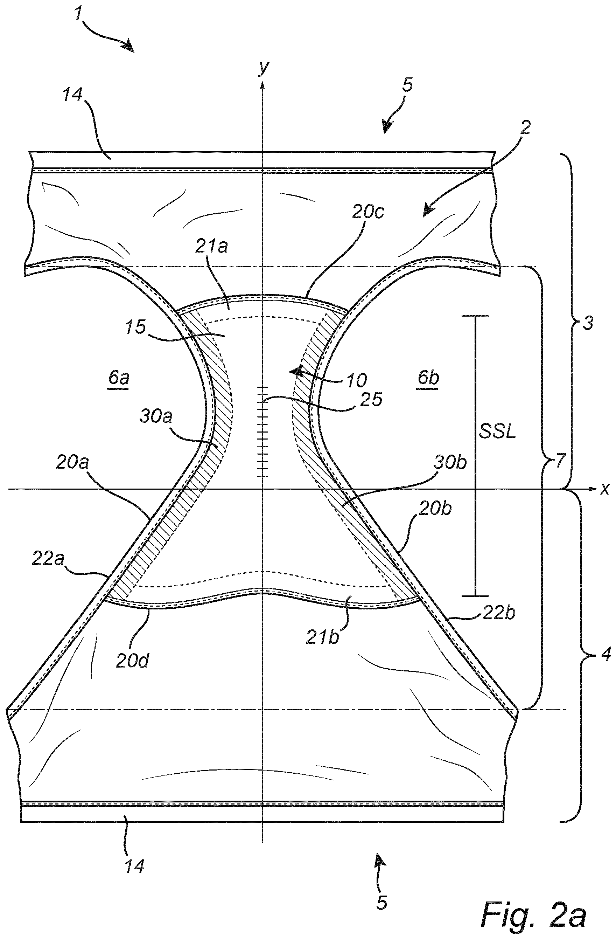

In view of the above, the central longitudinal axis y will be located centrally as seen over a total transversal width of the undergarment 1. The transversal axis x is to divide the undergarment 1 into the front side 3 and the back side 4. The location of the transversal axis x, i.e., the division between the front side 3 and the back side 4, may be determined by laying the complete undergarment 1 out with the back of the undergarment 1 towards a supporting surface and the front of the undergarment 1 towards the viewer as seen in Fig. 2a. The waist edge of the front of the undergarment 1 is to be superposed over the waist edge of the back of the undergarment 1. When the undergarment 1 is laid flat in this manner, the front side 3 of the undergarment 1 is towards the viewer, the back side 4 of the undergarment 1 is towards the supporting surface, and the transversal axis x extends between them, i.e., along a fold between the front side 3 and the back side 4 formed adjacent the crotch region 7 of the undergarment. As such, the front side 3 will be visible from the front of the user when the undergarment 1 is worn, and the back side 4 will be visible from the back of the user when the undergarment 1 is worn. As understood from the above, the transversal axis x may be a central transversal axis of the undergarment 1.

The undergarment of the Fig. 1 example comprises one fabric panel 2, which is cut to form the front side 3 and the back side 4, with the waist opening 5 and the leg openings 6a, 6b. However, in other variants, a plurality of fabric panels may be joined so as to form the front side 3, the back side 4 and the crotch region 7. For example, the undergarment 1 may comprise a front panel, a back panel and a crotch panel. The one or more fabric panels may all comprise the same fabric, giving the undergarment a unitary appearance, or the one or more fabric may comprise different fabrics, for example to provide aesthetically pleasing undergarments comprising e.g., lace fabric.

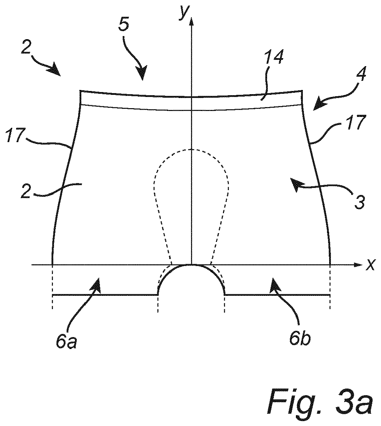

Depending on the cut of the one or more fabric panels 2, different undergarment styles may be formed. For example, as in the undergarment style shown in Fig. 1, the leg openings 6a, 6b may have a relatively high cut such as in a bikini style or a Brazilian style. In other examples, such as the undergarment style shown in Fig. 3a, the undergarment 1 may comprise leg portions extending beyond the crotch region 7 in a direction away from the waist opening 5 when the undergarment 1 is worn.

To join the front side 3 and the back side 4 so as to form the waist opening 5 and the leg openings 6a, 6b, a pair of side joints 17 may be provided, as in the illustrated example. The side joints 17 and any other joints joining the one or more fabric panels 2 may be conventional joints in the art, such as seams made by conventional adhesive and/or mechanical bonds such as conventional adhesive or stitching techniques.

As such, the absorbent undergarment 1 may be designed and manufactured in accordance with conventional sewing techniques. For example, the absorbent undergarment may comprise a waist band 14, arranged along the waist opening 5 of the absorbent undergarment 1. For example, the absorbent undergarment 1 may comprise edgings 22a, 22b arranged along the leg openings 6a, 6b.

As mentioned in the above, a central longitudinal axis y of the undergarment 1 is defined along the one or more fabric panels 2 of the undergarment 1 from the back side 4 and towards the front side 3. As such, when an undergarment 1 is seen from the front side 3 as in the example of Fig. 1, the central longitudinal axis y would be directed towards the waist opening 5 of the undergarment 1. However, when the undergarment 1 is seen from the back side 4 (not shown), the central longitudinal axis y would be directed away from the waist opening 5 of the undergarment 1.

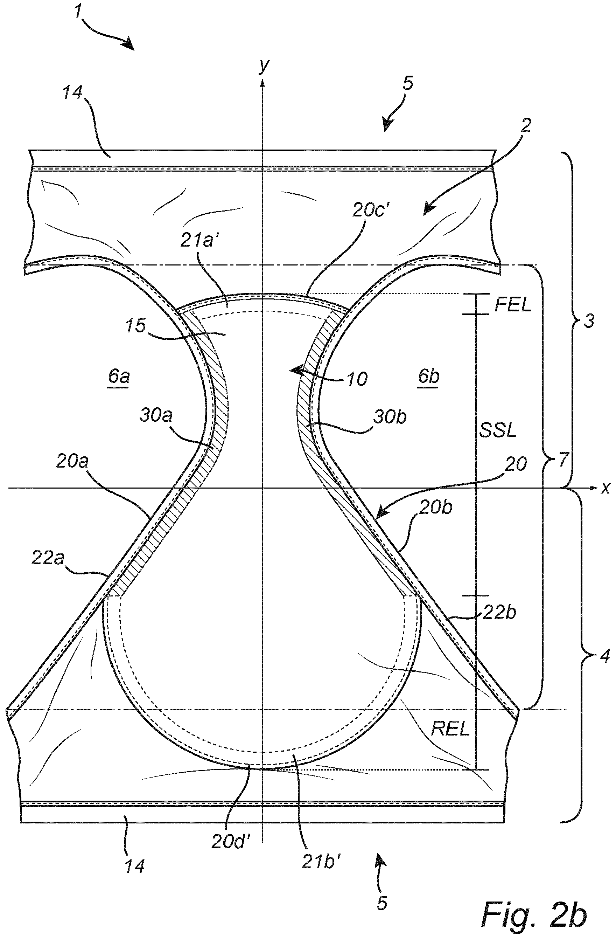

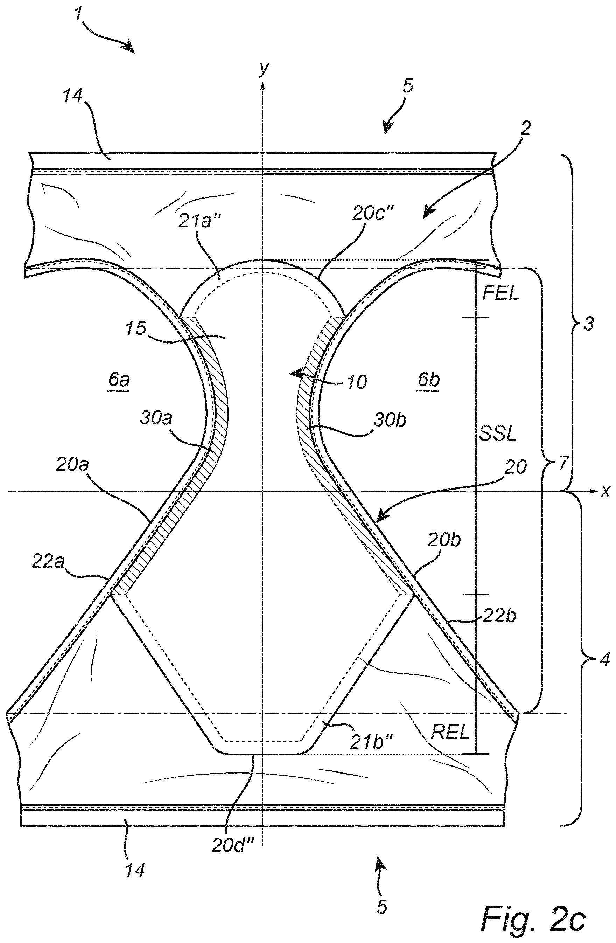

In Fig. 2a, the absorbent undergarment 1 of Fig. 1 is shown in a flat laid-out state, where the side joints 17 of the undergarment 1 are removed. As seen in this laid-out state, the central longitudinal axis y is defined along the one or more fabric panels 2 and in a direction from the rear of the undergarment 1 towards the front of the undergarment 1. In Fig. 2a, the absorbent undergarment 1 is shown from a wearer-facing side of the article 1. As illustrated in Fig. 1, the one or more fabric panels 2 will have an exterior side 9 facing away from the wearer, and an interior side 8 facing in a direction towards the wearer. Fig. 2 thus displays the undergarment 1 in a flat laid-out state as seen from the interior side 8.

As seen in Fig. 2a, the undergarment 1 forms leg openings 6a, 6b. A crotch region 7 is defined as being the portion of the undergarment 1 extending transversely between the leg openings 6a, 6b. As such, the crotch region 7 has a longitudinal extension corresponding to the longitudinal extension of the leg openings 6a, 6b, as indicated in Fig. 2a. It will be understood that depending on the style of the undergarment, i.e., the cut of the leg openings, the longitudinal extension of the crotch region 7 according to this definition may vary considerably.

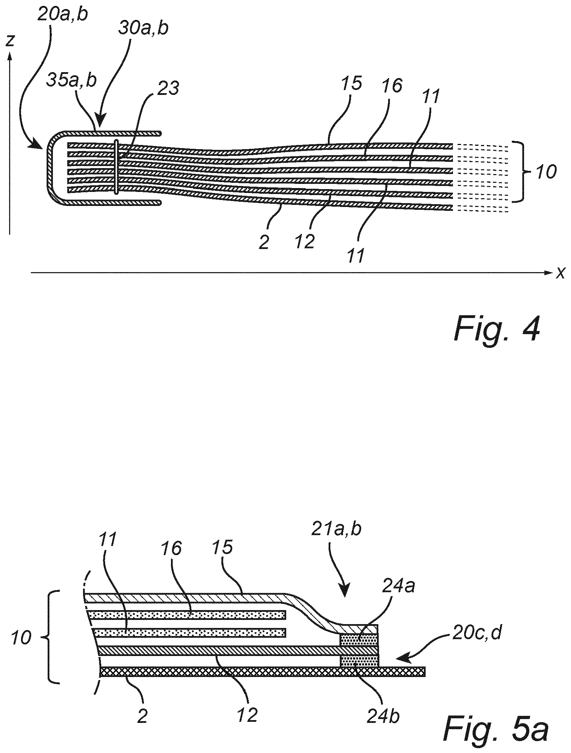

The undergarment 1 further comprises an absorbent assembly 10. As shown in e.g., Figs. 4a, 4b and 4c, the absorbent assembly 10 comprises a wearer facing top layer 15, a moisture barrier 12, and optionally one or more intermediate layers 11, 16 being superimposed between the wearer facing top layer 15 and the moisture barrier 12 along a height axis z perpendicular to the central longitudinal axis y and the transversal axis x. The layers 11, 12, 15, 16 of the absorbent assembly 10 will be further described in the below in relation to Figs. 4a, 4b and 4c.

As seen in Fig. 2a, in the illustrated undergarment 1, the absorbent assembly 10 is located on an interior side 8 of the at least one fabric panel 2. The absorbent assembly 10 is arranged in at least part of the crotch region 7 of the undergarment 1.

Although in some variants the absorbent assembly 10 may extend longitudinally beyond the crotch region 7 in a direction towards the front of the undergarment 1, in other variants, such as the variant illustrated in Fig. 2a, the absorbent assembly 10 extends no further than the crotch region 7 in the longitudinal direction towards the front side 3 of the undergarment 1.

The longitudinal extension of the absorbent assembly 10 towards the rear of the undergarment 1 may depend for example on the design of the undergarment 1. In some variants, such as in the illustrated variant of Fig. 2a, the absorbent assembly 10 may extend no further than the crotch region 7 in the longitudinal direction towards the rear region 4 of the undergarment 1.

As such, and as illustrated in the example of Fig. 2a, the absorbent assembly 10 may be confined to the crotch region 7, wherein the need for absorbance is immediately prevalent.

However, in other variants, the absorbent assembly 10 may extend beyond the crotch region 7 as illustrated e.g., in the variant of Fig. 2b. Further, other options may be available involving the absorbent assembly 10 alternatively or additionally extending beyond the crotch region 7 in a direction towards the front of the undergarment, as illustrated e.g., in the variant of Fig. 2c.

As exemplified by the illustrated undergarment 1, the absorbent assembly 10 forms a circumferential outer edge 20, forming a closed circumference of the absorbent assembly 10.

When the undergarment 1 is worn, the majority of a fluid insult, such as urine or menstrual fluids will contact a central crotch area of the undergarment 1, i.e., an insult region 25. It has been realized that due to the proximity of the leg openings 6a, 6b to the insult region 25, in combination with the wearer's movements, these areas of the undergarment 1 are more prone to leakage than other areas.

With "leg openings" it is herein referred to the portion of the undergarment 1 through which the leg will extend from the crotch region 7 of the undergarment 1. Thus, if the side joints 17 of the undergarment 1 is removed, as exemplified in Fig. 2a, and the crotch region 7 with the absorbent assembly 10 is laid out flat the leg openings 6a, 6b are understood to be the openings as seen in the plane of the transversal axis x and the longitudinal axis y through which the legs of the user will extend along an axis z, perpendicular to the transversal axis x and the longitudinal axis y when the article is worn. Thus, any portions of the undergarments 1 intended to extend along the leg of the wearer are disregarded when determining the extension of the leg openings 6a, 6b. I.e., when the complete undergarment 1 is in a flat state as illustrated in Fig. 1, any portions of the undergarment extending beyond the location of the transversal axis x in a direction away from the waist opening 5, are to be disregarded (e.g. may be cut off) when determining the extension of the leg openings 6a, 6b.

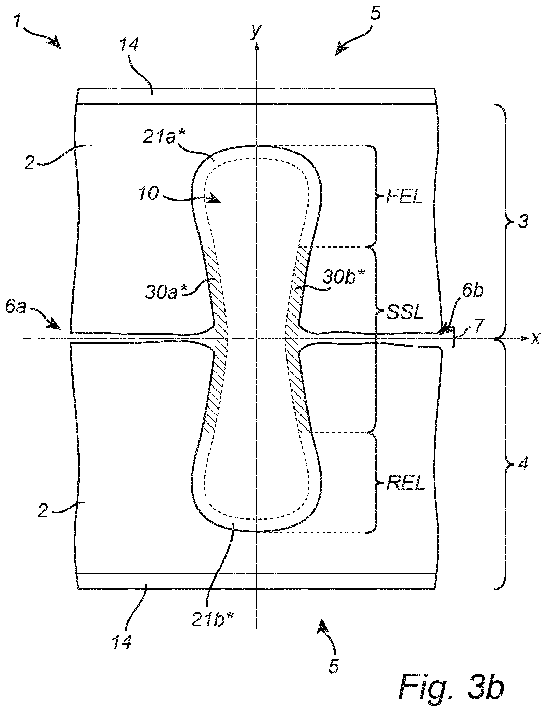

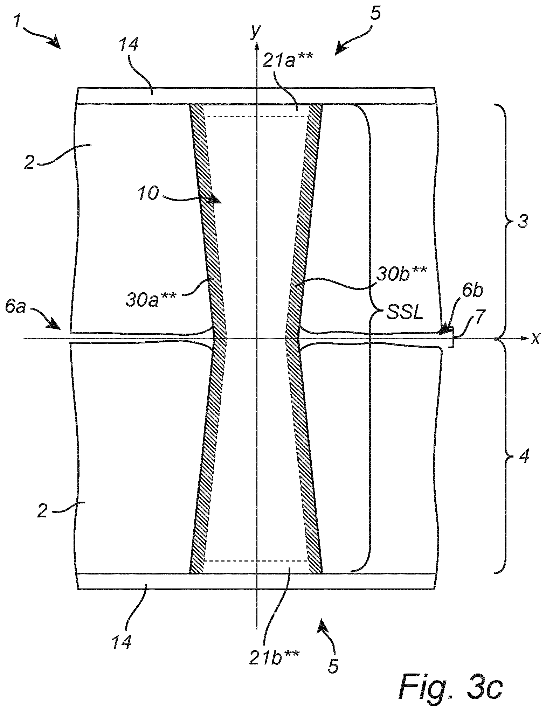

The absorbent assembly 10 in the undergarment 1 as disclosed herein, and as illustrated in the examples of Figs. 2a, 2b, 2c, 3b, and 3c, comprises one or more sealed edge sections 21a,a',a",a*,a**, 21b,b',b",b*,b**.

Figs. 5a-5c illustrate different examples of sealed edge sections 21a,a',a",a*,a**, 21b,b',b",b*,b**. In Figs. 5a-5c, the absorbent assembly 10 comprises a wearer facing top layer 15 facing the skin of the user and a liquid-impermeable moisture barrier 12. Two optional intermediate layers 11, 16, in this case two absorbent layers 11 and 16, are located between the wearer facing top layer 15 and the liquid-impermeable moisture barrier 12. The liquid-impermeable barrier 12 prevents liquid from leaking from the optional one or more intermediate layers 11 into the fabric panel 2. The liquid-impermeable barrier 12 may be in the form of a liquid-impermeable barrier layer 12 as illustrated in Fig. 5a. In other variants however, the liquid-impermeable barrier 12 may be a coating on the wearer facing side of a fabric panel 2 or on a garment facing side of an absorbent layer 11.

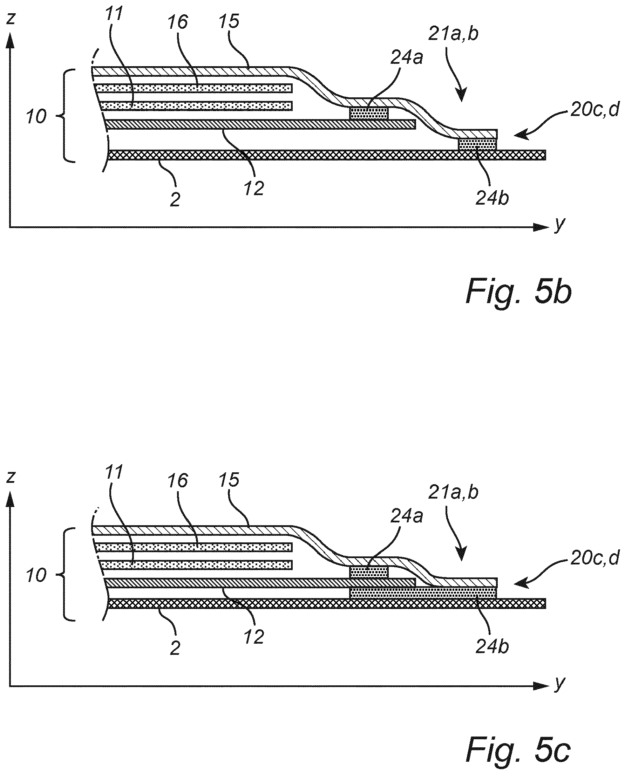

The different layers in the absorbent assembly 10 are joined in sealed edge sections 21a,a',a",a*,a**, 21b,b',b",b*,b** by fluid seals and/or fluid sealing joints of any kind as is conventionally known in the art. Figs 5a,5b and 5c show different, non-limiting examples of a schematic cross-sectional view of fluid seals 24a, 24b which may be comprised in a sealed edge section 21a,a',a",a*,a**, 21b,b',b",b*,b** as illustrated in for example Figs. 2a to 2c, or Figs. 3b to 3c.

Where the one or more intermediate layers 11, 16 does not extend into the circumferential margin, joining of the wearer facing top layer 15 and the moisture barrier 12 is accomplished by means of a first fluid seal 24a as seen in Fig. 5a. The first fluid seal 24a is arranged to extend between the bottom surface of the wearer facing top layer 15 and the top surface of the moisture barrier 12 at a distance from the circumferential outer edge of the one or more sealed edge sections. The fluid seal 24a provides a continuous sealing between the wearer facing top layer 15 and the moisture barrier 12 in the sealed edge sections.

In addition to the first fluid seal 24a, a second fluid seal 24b is arranged to extend between the bottom surface of the moisture barrier 12 and the top surface of the at least one fabric panel 2 along the circumferential outer edge 20 at a distance from the circumferential outer edge 20 in the one or more sealed edge sections 21a, 21b. The second fluid seal 24b provides additional attachment of the absorbent assembly 10 to the fabric panel 2, in addition to providing a further fluid seal along the one or more sealed edge sections 21a, 21b.

However, other variants are possible where the fabric panel 2 is not included in the seal created by the fluid seal 24b, but the absorbent assembly 10 is permanently attached to one or more fabric panels 2 of the undergarment 1 by other means, for example by a seam.

An advantage of joining the bottom surface of the wearer facing top layer 15 directly to the moisture barrier layer 12, without involving any of the intermediate layers 11, 16 is that flatter and pliable outer edge portions are provided allowing for more suitable fit and comfort to a user of the undergarment 1, while still hindering fluid leakage in a transversal direction x.

Fig. 5b is similar to Fig. 5a in that the one or more of the optional intermediate layers 11, 16 in the absorbent assembly 10 are arranged so as to not extend all the way to the circumferential outer edge 20 (as seen in a transversal and/or lateral direction) when joining of the wearer facing top layer 15 and the moisture barrier 12. A first fluid seal 24a is arranged to extend between the bottom surface of the wearer facing top layer 15 and the top surface of the moisture barrier 12 at a distance from the circumferential outer edge of the one or more sealed edge sections. The fluid seal 24a provides a continuous sealing between the wearer facing top layer 15 and the moisture barrier 12 in the sealed edge sections 21a, 21b.

Fig. 5b differs from Fig. 5a in that the wearer facing top layer 15 extends past the first fluid seal 24a (as seen in a transversal and/or lateral direction) and the bottom surface of the wearer facing top layer 15 is directly attached to a top surface of at least one fabric panel 2 by means of a second fluid seal 24b.

The wearer facing top layer 15 may extend beyond the moisture barrier 12 by a distance from 1 to 30 mm wherein the distance between the first fluid seal 24a and the outer edge of the wearer facing top may be 1-20 mm, preferably 1-10 mm, more preferably 3-10 mm.

Fig. 5c is similar to Figs. 5a and 5b in that the one or more of the optional intermediate layers 11, 16 in the absorbent assembly 10 are arranged so as to not extend all the way to the circumferential outer edge 20 (as seen in a transversal and/or lateral direction) when joining of the wearer facing top layer 15 and the moisture barrier 12. A first fluid seal 24a is arranged to extend between the bottom surface of the wearer facing top layer 15 and the top surface of the moisture barrier 12 at a distance from the circumferential outer edge of the one or more sealed edge sections. The fluid seal 24a provides a continuous sealing between the wearer facing top layer 15 and the moisture barrier 12 in the sealed edge sections 21a, 21b.

Similarly, to the variant in 5b, the wearer facing top layer 15 in variant 5c extends past the first fluid seal 24a (as seen in a transversal and/or lateral direction) and the bottom surface of the wearer facing top layer 15 is directly attached to a top surface of at least one fabric panel 2 by means of a second fluid seal 24b. However, in the variant of Fig. 5c, the second seal 24b may be broader than in in Fig. 5b and extends into an area underneath the first seal 24a. This gives additional fluid sealing in case some fluid should enter via the portion of the wearer facing top layer 15 extending to the outside of the first seal 24a.

Also, this may give a smoother outer edge since it diminishes the risk of the outer edge of the wearer facing top layer 15 wrinkling at the location of the second seal 24b.

Optionally, the second seal 24b extends all the way into the area, (or even beyond the area) underneath the first seal 24a. Thus, the second seal 24b may have a first portion forming a seal between the bottom side of the wearer facing top layer 15 and the top side of the fabric panel 2, and a second portion forming a seal between the bottom side of the moisture barrier 12 and the topside of the fabric panel 2.

The fluid seals 24a, 24b may, as exemplified in Fig. 2a, be arranged so as to substantially coincide with the circumferential outer edge 20 of the absorbent assembly 10.

In other variants, the fluid seals 24a, 24b may be arranged to extend along the circumferential outer edge 20 and at a distance from the circumferential outer edge 20.

In some variants, the distance may be constant, i.e., the one or more fluid seals 24a, 24b may generally follow the contour of the circumferential outer edge 20.

In yet other variants, the distance may vary, i.e., the one or more fluid seals 24a, 24b may have a shape differing more or less from the contour of the circumferential outer edge 20.

In either case, the one or more fluid seals 24a, 24b may advantageously extend relatively close to the circumferential outer edge 20, leaving the majority of the absorbent assembly 10 available for intake and distribution of bodily fluids. Thus, the distance may be for example less than 5 mm. For example, the distance may be less than 2 mm or less than 1mm.

Optionally, the fluid seals 24a, 24b may comprise one or more fluid sealing joints. As in the illustrated example, each fluid seal 24a, 24b comprises a fluid sealing joint. For example, such a fluid sealing joint may be accomplished by a fluid sealing adhesive joint or a fluid sealing welded joint, such as a heat welded joint or an ultrasonic welded joint.

In the case of a welded sealing joint, the fluid seal may be formed from melted material. For example, the sealing joint may comprise a hot-melt adhesive. In another example, the sealing joint may comprise melted material from e.g., the layers to be joined together, such as an ultrasound sealing joint or heat-sealing joint.

The fluid seal may be a double-sided adhesive tape. In some variants both the first and second fluid seals 24a, 24b may be a double-sided adhesive tape.

In some variants, the bottom surface of the wearer facing top layer 15 and the top surface of the moisture barrier 12 may be directly joined by at least one of the fluid sealing joints 24a.

In other variants, the bottom surface of the moisture barrier layer 12, and the top surface of the fabric panel 2 may be joined by a fluid sealing joint 24b.

In other variants, the bottom surface of the wearer facing top layer 15, and the top surface of the fabric panel 2 may be joined by a fluid sealing joint 24b.

In some examples, such as those illustrated in the drawings, the circumferential outer edge 20 of the absorbent assembly 10 further comprises sealed portions not being sealed edge sections as defined herein. Such sealed portions may be made using various other sealing arrangements, for example so as to provide an even higher resistance to leakage than the sealed edge sections. For example, and as illustrated in the drawings, the circumferential outer edge 20 of the absorbent assembly 10 may comprise one or more sealed assembly side portions 30a, 30b located opposite to each other and on each side of the longitudinal axis y.

For example, and as in the variants of the drawings, in each sealed assembly side portion 30a, 30b, a top surface of the wearer facing top layer 15 may be sealingly attached to the moisture barrier 12 or to the one or more fabric panels 2. Each sealed assembly side portion 30a, 30b may extend longitudinally along at least a part of the crotch region 7 of the undergarment 1. The sealed assembly side portions 30a, 30b may comprise fluid sealing members 35a, 35b being arranged so as to hinder fluid leakage from the absorbent assembly 10 to the outside of the circumferential outer edge 20 via the sealed assembly side portion 30a, 30b in the crotch region 7. A fluid sealing member 35a, 35b is in this context a fluid seal providing a continuous sealing along the sealed assembly side portion 30a, 30b and between the wearer facing top layer 15 and the moisture barrier 12. The fluid sealing member 35a, 35b in the sealed assembly side portions 30a, 30b may e.g., be provided by a sealing strip 35a, 35b arranged to be folded over the outer circumference of the wearer facing top layer 15 and the moisture barrier 12 as will be further explained in connection with Fig. 4 below.

Fig. 4 illustrates an example of an absorbent assembly 10 comprising a wearer facing top layer 15, a moisture barrier 12, and optionally one or more intermediate layers 11, 16 being superimposed between the wearer facing top layer 15 and the moisture barrier 12 along a height axis z perpendicular to the central longitudinal axis y and the transversal axis x. In Fig. 4, the layers 11, 12, 15, 16 are illustrated as being spaced from each other for better visibility. However, in practice the layers will be superimposed over one another, each layer contacting the neighboring upper layer and/or lower layer as seen in the height direction z.

In an absorbent assembly 10 comprising one or more of the optional intermediate layers 11, 16, all of the layers of the absorbent assembly 10 may, at least in some portions where a greater need for absorption of fluids is required, extend to the circumferential outer edge 20 of the absorbent assembly 10 . This may e.g., be the case in the sealed assembly side portions 30a,a*,a**, 30b,b*,b** extending longitudinally along the leg openings 6a, 6b where the majority of a fluid insult, such as urine or menstrual fluids, will contact a central crotch area of the undergarment 1. As such, the absorbent assembly 10 may comprise the wearer facing top layer 15 and the moisture barrier 12 with one or more intermediate layers 11, 16 along a sealed side margin length SSL. Here, fluid sealing members 35a, 35b comprising sealing strips arranged to be folded over the circumferential edge of the absorbent assembly 10 including all layers is advantageously used (see Fig. 4). The fluid sealing member 35a, 35b may be arranged to extend from a top side of the wearer facing top layer 15, over the outer circumferential edges of the wearer-facing top layer 15, the optional one or more intermediate layers 16, 11, and the barrier layer 12. As in the example of Fig. 4, the fluid sealing member 35a, 35b may extend also over an outer edge of the one and more fabric panels 2 and to a bottom side of the one or more fabric panels. As such, the fluid sealing member 25a, 25b may attach the absorbent assembly 10 to the one or more fabric panels 2. In other variants (not shown) the fluid sealing member 35a, 35b may extend from a top side of the wearer facing top layer 15, over the outer circumferential edges of the wearer facing top layer 15, the optional one or more intermediate layers 16, 11, and the barrier layer 12, and to a bottom side of the barrier layer 12.

Optionally, a mechanical joint, such as e.g., a seam can join the layers 15, 12, 16, 11 together inside their peripheral edges.

As such, the fluid sealing member 35a, 35b, which may for example be an elastic tape, forms a continuous sealing between the top side of the wearer facing top layer 15 and the moisture barrier 12, for example to the bottom side of the moisture barrier 12 or to the bottom side of the one or more fabric layers 2, so as to hinder fluid leakage.

Each of the fluid seals 20a, 20b in the sealed assembly side portions 30a, 30b may extend continuously over a longitudinal side sealing length SSL, as seen along the longitudinal axis y.

To provide sufficient sealing, the longitudinal side sealing length SSL may for example be at least 5 cm, such as at least 10 cm.

The longitudinal side sealing length SSL may for example be less than 45 cm, such as less than 35 cm.

As such, the longitudinal side sealing length SSL may be selected so as to be suitable for providing sufficient hinder to fluid leakage via the sealed assembly side portions 30a,a*,a**, 30b,b*,b**.

In some embodiments the sealing member 35a, 35b does not extend over the outer peripheral edge of the fabric panel 2. Instead, the absorbent assembly 10 including the sealing member 35a, 35b may be attached to the fabric panel 2 by an edging 22a, which extends over the sealing member 35a, 35b and the outer peripheral edge of the fabric panel 2. As such, an edging 22a may be used to attach an absorbent assembly 10 as described in any of the examples or illustrations herein to a fabric panel 2.

It is to be understood, that although the examples illustrated in the drawings all comprise sealed assembly side portions 30a, 30b, the present disclosure encompasses also variants without any such sealed assembly side portions 30a, 30b.

As mentioned above, areas of the absorbent assembly 10 less prone to leakage and/or have higher requirements for pliability, may advantageously be provided with fluid seals that are thinner (i.e., flatter) and more pliable than seals in the more leakage prone regions. Thus, in the sealed edge sections 21a,a',a",a*,a**, and 21b,b',b",b*,b** provided e.g., in areas of the absorbent assembly 10 facing the front 3 and/or back 4 of the undergarment 1, one or more of the intermediate layers 11, 16 (if present) may extend over less than the complete area of the absorbent assembly 10. In other words, in selected areas of the absorbent assembly 10, the assembly comprises less than all of the layers of the absorbent assembly.

For example, selected areas of the absorbent assembly 10 may comprise only the wearer facing top layer 15 and the moisture barrier 12. This may be advantageous for example in areas of the absorbent assembly 10 where a lesser need for absorption of fluid prevails and/or where better fit and forming of the undergarment 1 to the user's body is required.. For example, when the absorbent assembly 10 comprises a sealed front and/or rear edge section, the transversal areas of absorbent assembly 10 corresponding to the sealed front and/or rear edge sections may be selected areas comprising only the wearer facing top layer 15 and the moisture barrier 12. As such, the absorbent assembly 10 may comprise the wearer facing top layer 15 and the moisture barrier 12 with no intermediate layers along a front margin length FEL and/or a rear margin length REL.

In some variants, such as the one illustrated in Fig. 2a, in each sealed assembly side portion 30a, 30b, at least a part of the circumferential outer edge 20 of the absorbent assembly follows 10 the contour of a respective leg opening 6a, 6b of the undergarment 1. In the variant of Fig. 2a, throughout each sealed assembly side portion 30a, 30b, the circumferential outer edge 20 of the absorbent assembly follows the contour of a respective leg opening 6a, 6b.

However, in other variants (not shown), in each sealed assembly side portion 30a, 30b, a part of the circumferential outer edge 20 of the absorbent assembly may follow the contour of a respective leg opening 6a, 6b, and another part of the circumferential outer edge 20 may deviate from the contour of a respective leg opening 6a, 6b.

Although the sealed assembly side portions 30a, 30b are efficient to hinder leakage, it has now been realized that sealed assembly side portions may become stiff and bulky. Thus, it is proposed herein to provide sealed edge sections which may provide a flatter and more pliable seal, while providing sufficient sealing. The flatter and more pliable seals in the sealed edge sections provide the washable and reusable undergarment 1 with an improved aesthetics, fit and comfort compared to absorbent assemblies 10 provided with e.g., fluid sealing members arranged to be folded over the entire outer circumferential edge 20. Furthermore, and as will be described in further detail below, sealed edge sections provide for easy manufacturing while still achieving a leakproof circumferential outer edge 20.

In some examples, such as those illustrated in the drawings, sealed edge sections 21a, 21b are combined with sealed assembly side portions 30a, 30b. For example, the sealed assembly side portions 30a, 30b may be arranged generally along the leg openings of the undergarment (side edges of the absorbent assembly), where the requirements for pliability may be less, whereas sealed edge sections 21a, 21b may be arranged at e.g. a front edge and/or a rear edge of the absorbent assembly, where the requirements for pliability may be greater.

Also , although naturally it is desired to avoid leakage of body fluids from the absorbent assembly 10 as a whole, it has now been realized that for some portions of the circumferential outer edge 20, the risk of leakage occurring is nevertheless less. For example, portions of the circumferential outer edge 20 that are relatively distanced from the insult region 25 of the undergarment 1 may not be that prone to leakage since the fluid received at the insult region 25 needs to be distributed over a relatively long distance in order to reach portions close to the circumferential outer edge 20.

Accordingly, portions of the circumferential outer edge 20 which are deemed prone to less leakage may advantageously be provided with sealed edge sections 21a, 21b.

Other examples (not shown in the drawings) are however possible, wherein the absorbent assembly comprises sealed edge sections 21a, 21b but no sealed assembly side portions 30a, 30b as described herein. For example, the absorbent assembly may comprise sealed edge sections 21a, 21b around the entire outer circumferential edge of the absorbent assembly. Also, other examples are possible where sealed edge sections 21a, 21b are combined with any other type of sealing arrangement along a portion or portions of the circumferential outer edge of the absorbent assembly.

Each sealed edge section provides a continuous sealing along the sealed edge section, and between the wearer facing top layer 15 and the moisture barrier 12 so as to hinder fluid leakage from the absorbent assembly 10 to the outside of the circumferential outer edge 20 via the sealed edge section. Continuous sealing of the sealed edge sections is provided by sealingly attaching a bottom surface of the wearer facing top layer 15 to a top surface of the moisture barrier 12, and directly attaching a bottom surface of the moisture barrier 12 or a bottom surface of the wearer facing top layer 15 to a top surface of at least one fabric panel 2.

Advantageously, in some variants, at least a portion of the circumferential outer edge 20 forms a sealed front edge section 21a of the absorbent assembly 10 and faces the front 3 of the undergarment 1. The sealed front edge section 21a will provide continuous sealing along the sealed front edge section 21a, and between the wearer facing top layer 15 and the moisture barrier 12 so as to hinder fluid leakage from the absorbent assembly 10 to the outside of the circumferential outer edge 20 facing the front 3 of the undergarment 1.

Advantageously, in some variants at least a portion of the circumferential outer edge 20 forms a sealed rear edge section 21b of the absorbent assembly 10 and faces the back 4 of the undergarment 1. The sealed rear edge section 21b will provide continuous sealing along the sealed rear edge section 21b, and between the wearer facing top layer 15 and the moisture barrier 12 so as to hinder fluid leakage from the absorbent assembly 10 to the outside of the circumferential outer edge 20 facing the back 4 of the undergarment 1.

In some variants, the circumferential outer edge 20 of the absorbent assembly 10 comprises a front sealed edge section 21a facing the front 3 of the undergarment 1 and a back sealed edge section 21b facing the back 4 of the undergarment 1.

Thus, as will be understood by the example embodiments described herein, in some variants, the one or more sealed assembly side portions 30a, 30a, and the one or more sealed edge sections 21a, 21b may be distributed along the circumferential outer edge 20 such that complete, or substantially complete fluid sealing of the absorbent assembly 10 is provided to ensure a well-functioning absorbent undergarment 1, while at the same time providing desirable comfort and fit.

For example, sealed assembly side portions 30a, 30b may be arranged in pairs such that the sealed assembly side portion 30a, 30b of each pair is located opposite to each other and on each side of the longitudinal axis y. Similarly, the sealed edge sections 21a, 21b may be arranged in pairs such that the sealed edge section 21a, 21b of each pair is located opposite to each other and on each side of the transversal axis x.

For example, and as illustrated in the variant of Fig. 2a, the circumferential outer edge 20 of the absorbent assembly 10 may include a pair of sealed assembly side portions 30a, 30b provided with a fluid sealing member 35a, 35b (see Fig. 4) located opposite to each other and on each side of the longitudinal axis y. Each sealed assembly side portion 30a, 30b, extends longitudinally along at least a part of the leg openings 6a, 6b in the crotch region 7 of the undergarment 1.

The circumferential outer edge 20 of the absorbent assembly 10 further includes a pair of sealed edge sections 21a, 21b located opposite to each other and on each side of the transversal axis x such that a sealed front edge section 21a faces the front 3 of the undergarment 1, and a sealed rear edge section 21b faces the back 4 of the undergarment 1 as seen in Fig. 2a.

As mentioned above, by sealingly attaching a bottom surface of the wearer facing top layer 15 to a top surface of the moisture barrier 12, and directly attaching a bottom surface of the moisture barrier 12 or a bottom surface of the wearer facing top layer 15 to a top surface of at least one fabric panel 2, continuous sealing is provided along the sealed edge section 21a, 21b and between the wearer facing top layer 15 and the moisture barrier 12. The sealed edge sections 21a, 21b will thus efficiently hinder fluid leakage from the absorbent assembly 10 to the outside of the circumferential outer edge 20 via the sealed edge sections 21a, 21b.

The pair of sealed assembly side portions 30a, 30b together with the pair of sealed front and rear edge sections 21a, 21b will provide continuous sealing along the entire circumferential outer edge 20 of the absorbent assembly 10, so as to hinder fluid leakage from the absorbent assembly 10 to the outside of the circumferential outer edge 20 of the absorbent assembly 10.

By providing an absorbent assembly 10 having a pair of sealed assembly side portions 30a, 30b together with a pair of sealed edge sections 21a, 21b arranged around the circumferential outer edge 20 of the absorbent assembly 10, leakage of the fluid from the insult region 25 inside the absorbent assembly 10 to the outside of the circumferential outer edge 20 can be prevented.

Thus, as will be understood by the example embodiments described herein, the one or more sealed assembly side portions 30a, 30a, and the one or more sealed edge sections 21a, 21b may be distributed along the circumferential outer edge 20 such that complete, or substantially complete fluid sealing of the absorbent assembly 10 is provided to ensure a well-functioning absorbent undergarment 1, while at the same time providing desirable comfort and fit.

Thus, for example, and as illustrated in Fig. 2a, the circumferential edge 20 may comprise a rear edge portion 20d directed towards the back 4 of the undergarment 1, a front edge portion 20c directed towards the front 3 of the undergarment 1, and side edge portions 20a, 20b, directed towards the leg openings 6a, 6b of the undergarment. For example, and as illustrated in the variant of Fig. 2a, the side edge portions 20a, 20b may interconnect the front edge portion 20c and the rear edge portion 20d, so as to form the entire circumferential edge 20. Similarly, the sealed assembly side portions 30a, 30b may, as exemplified in Fig. 2a, extend along the side edge portions 20a, 20b and interconnect a sealed front edge section 21a extending along the front edge portion 20c and a sealed rear edge section 21b extending along the rear edge portion 20d.

The shape of the absorbent assembly 10 may be adapted to various absorption needs and to various designs of the undergarment 1, so as to provide sufficient absorption and satisfactory fit.

For example, the side edge portions 20a, 20b may comprise concave portions, as seen towards the central longitudinal axis y. For example, as in the illustrated variant of Fig. 2a, the entire side edge portions 20a, 20b are concave towards the central longitudinal axis y, i.e., curving inwards towards the central longitudinal axis y. Thus, the absorbent assembly 10 may be adapted to fit between the wearer's legs.

The front edge portion 20c of the absorbent assembly may similarly be shaped in view of the need for comfort and fit of the undergarment 1. In some variants, such as in the illustrated undergarment 1, the front edge portion 20c may be convex from the transversal axis x, i.e., curving away from the transversal axis x. As illustrated in Fig. 2a, at the location of the central longitudinal axis y, the front edge 20c may thus protrude towards the front of the undergarment 1 beyond the extension of the side edge portions 20 a, 20b of the absorbent assembly 10. This may be beneficial for the fit of the undergarment to the body and contribute to the avoidance of unwanted creases in the fabric panels 2.

Also, the rear edge portion 20d of the absorbent assembly 10 may be shaped in view of the need for comfort and fit of the undergarment 1. As illustrated in Fig. 2a, the rear edge portion 20d may for example be concave towards the transversal axis x, i.e., curving towards the central transversal axis x. In other variants, the rear edge portion 20d may protrude towards the rear of the undergarment 1 beyond the extension of the side edge portions 20a, 20b of the absorbent assembly 10.

Fig. 2b illustrates another variant of an undergarment 1. Features which are similar to features described in relation to Fig. 2a in the above are denoted with the same reference number. The description of the features made in relation to the variant of Fig. 2a applies equally to the variant of Fig. 2b.

The absorbent assembly 10 illustrated in Fig. 2b comprises a pair of sealed assembly side portions 30a, 30b, as described in relation to Fig. 2a.

Further, and as illustrated in Fig. 2b, the circumferential outer edge 20 of the absorbent assembly 10 may form a rear edge portion 20d' extending longitudinally from the rearmost longitudinal end of the sealed assembly side portions 30a, 30b towards the back portion 4 of the undergarment 1, to a rearmost end of the absorbent assembly 10. The rear edge portion 20d' may comprise one or more sealed edge sections 21 b'. For example, and as illustrated in Fig. 2b the one or more sealed edge sections 21b' of the absorbent assembly 10 comprises the rear edge portion 20d', i.e., the absorbent assembly 10 comprises a sealed rear edge section 21b'.

Thus, there is provided an undergarment 1 with a sealed rear edge section 21b' extending rearwardly of the sealed assembly side portions 30a, 30b. As such, this may be desired e.g., for forming undergarments 1 particularly suitable for nighttime use. By ensuring that the rear edge portion 20d' is a sealed rear edge section 21 b', the outer shape and extension of the absorbent assembly 1 in the rear edge portion 20d', a comfortable fit and substantially invisible rear edge portion 20d' of the absorbent assembly 10 is achieved due to the flat and pliable seal provided in the sealed rear edge section 21b'.

For example, the sealed rear edge section 21b' may have a Rear Edge Length (REL) along the longitudinal axis y. The Rear Edge length may for example be at least 2 cm, such as at least 4 cm or at least 10 cm. The Rear Edge Length could for example be less than 40 cm, such as less than 30 cm. The Rear Edge Length may be in the range from 2 cm to 40 cm, such as from 4 cm to 30 cm.

Thus, for example, an undergarment 1 may be provided wherein the sealed side length (SSL) of the sealed assembly side portions is between 5 and 35 cm, and the Rear Edge Length (REL) is at least 2 cm, such as between 4 and 30 cm.

In some variants, such as illustrated in Fig. 2b, the rearmost end of the absorbent assembly 10 may be arranged at a distance from a rear waist end of the undergarment 1.

For example, the rearmost end of the absorbent assembly 10 may be arranged at a distance of at least 2 cm, such as at least 4 cm or at least 6 cm from the rear waist end of the undergarment 1.

As exemplified in Fig. 2b, the absorbent assembly 10 may for example extend beyond the crotch region 7 of the undergarment 1 in a direction towards the back 4 of the undergarment 1.

Fig. 2c illustrates yet another variant of an undergarment 1. Features which are similar to features described in relation to Fig. 2a or Fig. 2b in the above are denoted with the same reference number, and the description of the features made in relation to the variant of Fig. 2a or Fig. 2b applies equally to the variant of Fig. 2c.

As described with reference to Fig. 2b, in the variant of Fig. 2c, the circumferential outer edge 20 of the absorbent assembly 10 may form a rear edge portion 20d" located in the portion of the absorbent assembly 10 extending longitudinally from the rearmost longitudinal end of the sealed assembly side portions 30a, 30b towards the back portion 4 of the undergarment 1, to a rearmost end of the absorbent assembly 10. The rear edge portion 20d" may comprise one or more sealed rear edge sections. For example, and as illustrated in Fig. 2c the one or more sealed edge sections 21 of the absorbent assembly 10 comprises the rear edge portion 20d", i.e., the rear edge portion 20d" is a sealed rear edge section 21 b".

Further, as in the variant of Fig. 2c, the circumferential outer edge 20 of the absorbent assembly 10 may form a front edge portion 20f located in the portion of the absorbent assembly 10 extending longitudinally from the frontmost longitudinal end of the sealed assembly side portions 30a, 30b to a frontmost end of the absorbent assembly 10. The front edge portion 20f may comprise one or more sealed edge sections 21a". For example, and as illustrated in Fig. 2c, the one or more sealed front edge sections 21a"of the absorbent assembly 10 comprises the front edge portion 20f, i.e., the front edge portion 20f is a sealed front edge section 21a".

As understood from the examples of Figs 2b and 2c, the provision of a sealed front edge section 21a" and/or a sealed rear edge section 21b" provides for flexibility when selecting the shape and extension of the front and/or rear edge portions. For example, and as illustrated in the example of Fig. 2b, the contour of the front and/or rear edge portion 20c", 20d" may comprise rounded shapes, e.g., a bulb shape. In other examples, such as illustrated in Fig. 2c, the contour of the front and/or rear edge portions 20c", 20d" may comprise straight shapes such as lines and/or corners.

Fig. 3a illustrates yet another variant of an undergarment 1, as shown when the complete undergarment 1 is laid out in a flat state on a support surface. Features which are similar to features described in relation to Fig. 1 and Figs 2a to 2c in the above are denoted with the same reference number, and the description of the features made in relation to the variant of Fig. 1 and Figs 2a to 2c applies equally to the variant of Fig. 3a and 3b. For simplicity, Figs 3a to 3c are shown with lesser detail than Figs. 1 and 2a to 2c. However, the skilled person will understand that the omitted details such as the location of the sealed assembly side portions 30a*, 30a**, 30b* and 30** and sealed edge sections 21a*, 21a**, 21b* and 21b**, may equally be applied and adapted to the variants of Figs. 3a to 3c.

The undergarment 1 of Fig. 3a differs from the undergarments illustrated in Figs. 1 and 2a to 2c primarily by that the undergarment 1 is of a style having legs extending beyond the transversal axis x in a direction away from the waist opening 5, when the complete undergarment is laid out as in Fig. 3a. As mentioned in the above, extending leg portions are to be disregarded when determining the leg openings 6a, 6b as defined herein. Thus, In Figs. 3b and 3c, an undergarment 1 of the style as illustrated in Fig. 3a, is shown with any leg portions extending beyond the transversal axis x in a direction away from the waist opening 5 cut off from the undergarment 1.

Turning to Fig. 3b, which illustrates the undergarment 1 of Fig. 3a in a cut-open state similar to what was described in Figs. 2a to 2c, it is found that for this type of style, when determining the extension of the leg openings 6a, 6b, these openings may have a relatively short extension along the longitudinal axis y. As such, by definition, the crotch region 7 extending between the leg openings may similarly be relatively short.

The absorbent assembly 10 may, as illustrated in the example of Fig. 3b, extend beyond the crotch region 7 towards the front 3 and the back 4 of the undergarment 1. As described in relation to the other examples, the absorbent assembly 10 may for example comprise a pair of sealed assembly side regions 30a*, 30b*, a sealed front edge region 21a* and/or a sealed rear edge region 21b*. For example, and as in the variant of Fig. 3b, the absorbent assembly 10 may comprise a sealed front edge region 21 a* and a sealed rear edge region 21 b*. The extension and shape of the front and/or rear edge regions 21a*, 21b* may be freely selected, and may, for example, as in Fig. 3b be approximately similar between the front and rear margin regions 21a*, 21b*. As mentioned in the above, the rearmost end and/or the frontmost end of the absorbent assembly 10 may be arranged at a distance from a waist edge at the waist opening 5 of the undergarment 1.

Fig. 3c illustrates yet another variant of an undergarment 1. The general style of the undergarment in Fig. 3c is similar to the one described in relation to Fig. 3a and 3b. Features which are similar to features described in relation to Fig. 1 and Figs 2a to 2c, or Figs. 3a to 3b in the above are denoted with the same reference number, and the description of the features made in relation to the variant of Fig. 1 and Figs 2a to 2c, or Figs. 3a to 3b applies equally to the variant of Fig. 3c.

For example, and as illustrated in Fig 3c, the absorbent assembly 10 may extend over essentially the entire front side 3 and/or rear side 4 of the undergarment 1. As shown in the example of Fig. 3c, the rear end of the absorbent assembly 10 may coincide with a rear waist end of the undergarment 1 as formed by the one or more fabric panels 2. Similarly, and as in the example of Fig. 3c, the front end of the absorbent assembly 10 may coincide with a front waist end of the undergarment 1.

As illustrated in Fig. 3c, the absorbent assembly 10 may comprise sealed assembly side portions 30a**, 30b**, which extend longitudinally over the entire front side 3 and/or back side 4 of the undergarment 1. Thus, the side sealing length SSL corresponds to the total longitudinal extension of the absorbent assembly 10. The absorbent assembly may further comprise a sealed front edge section 21a** and/or a sealed rear edge section 21b**. The sealed front and/or rear edge sections 21a**, 21b** may coincide with the front waist edge and/or the rear waist edge as formed by the one or more fabric panels 2, and optionally be provided with a waist band 14.

In other variants, the side sealing length SSL may be less than the total longitudinal extension of the absorbent assembly, as discussed in the above in relation to the variants of Figs. 1 to 2c. Again, features and explanations made in relation to Figs. 1 and 2a to 2c are applicable also to styles such as illustrated in Figs 3a to 3c.

The wearer facing top layer 15 may be constructed of any suitable fabric, including naturally derived fibers, synthetic fibers, or a blend or a mixture of naturally derived and/or synthetic fibers. The materials used for construction of the wearer facing top layer should be soft and non-irritating to the skin and be readily penetrated by any body fluids.

According to the present disclosure, the wearer facing top layer should be washable. The wearer facing top layer may comprise between 20% and 100% naturally derived fibers, more preferably between 40% and 100% naturally derived fibers and most preferably between 60% and 100% naturally derived fibers. The top skin-facing layer 15 comprises a water-permeable material thus allowing the body fluids to migrate to the underlying absorbent layer or layers 11. The wearer facing top layer 15 may have a basis weight of 80-200 gsm.

In some variants (not illustrated), a wicking layer 16 may be provided underneath the wearer facing top layer 15. The wicking layer 16 has wicking features to allow the moisture to spread away from the wearer and into the absorbent layer or layers 11. Further, wicking enables an efficient spread of the moisture therefore allowing the moisture to be received in a wider area of the underlying one or more absorbent layers 11. This feature is particularly relevant for users suffering from stress incontinence as spurts of urine may cause the absorbent layer 11 to be saturated rapidly at the point of impact. By spreading the volume of the fluid over a wider receiving area in the absorbent layer 11, the wicking features of the wicking layer 16 increase the overall absorptive capacity of the absorbent layer. The wicking layer 16 may be constructed of any suitable fabric, including naturally derived fibers or mixtures thereof, synthetic fibers or mixtures thereof and/or a blend or a mixture of naturally derived and/or synthetic fibers. The basis weight of the wicking layer may be 180-250 gsm.

The absorbent assembly 10 of the absorbent undergarment 1 may optionally contain one or multiple absorbent layers 11, capable of absorbing liquid and releasing the liquid during laundering. Release of the absorbed liquid is beneficial as it enables the absorbent undergarment 1 to be restored for reuse. Reusable absorbent undergarments provide a more sustainable alternative to the commonly used disposable hygiene articles that are not intended to be re-used.

The absorbent layer or layers 11 may comprise any material capable of absorbing fluid, such as natural or synthetic fibers The odor treatment may be silver ions, copper ions and zeolites or Polyhexamethylene biguanide, PHMB.

The absorbent layer or layers 11 may also comprise a material having an open cell porous structures such as high loft or synthetic fibers having reservoir properties. The absorbent layer may comprise between 20% and 100% naturally derived fibers, more preferably between 40% and 100% naturally derived fibers and most preferably between 60% and 100% naturally derived fibers. The basis weight of each absorbent layer 11 may be 200-350 gsm. There may be one or more absorbent layers 11 in the absorbent assembly 10. For example, as in some of the illustrated variants, the absorbent assembly 10 may comprise two absorbent layers 11.

The liquid-impermeable barrier 12 may comprise any suitable material or combinations of material that prevents liquid from migrating from the absorbent assembly 10 to the fabric layer 2. The liquid-impermeable barrier 12 may, as in the illustrated variant, be a liquid-impermeable barrier layer having inherent hydrophobic properties, or that has been treated to become hydrophobic. The liquid-impermeable barrier layer 12 may comprise a microporous polymer film, or laminates of polymer films and nonwoven materials may also be used. Alternatively, the liquid-impermeable barrier 12 may be a coating of a moistureimpermeable material. The coating may be provided for example on the surface facing away from the wearer of an absorbent layer 11, or on the surface facing towards the wearer of a fabric panel 2.

The liquid-impermeable barrier 12 is preferably breathable, to allow vapor to escape from the absorbent undergarment 1, while preventing liquid from passing through the fabric layer. Further, the liquid-impermeable barrier 12 may be constructed of an elastic material, thus providing the absorbent assembly 1 with the required flexibility to adapt to the user's anatomy and movements. This improved fit increases the wearer's comfort during use and helps to prevent leakage from migrating through the undergarment's leg openings 6a, 6b.

The fabric of the one or more fabric panels 2 may be constructed of any suitable fabric, including naturally derived fibers and mixtures thereof, synthetic fibers or mixtures thereof or of a blend or a mixture of naturally derived and/or synthetic fibers. The fibers may be recycled fibers. The fabric may comprise a stretchable fabric so that the absorbent undergarment can provide a firm fit while at the same time adapting to the wearer's movements thus preventing any leakage from migrating through the leg openings and keeping the absorbent assembly in place. The fabric is preferably breathable to allow vapor to escape from the wearer's skin and from the absorbent assembly.

The disclosure may be varied within the scope of the appended claims. For example, the materials and dimensions used for the different layers forming the absorbent assembly may be varied, as indicated above.

## Claims

1. A washable and reusable undergarment (1), comprising one or more fabric panels (2) forming a front side (3) and a back side (4) being joined such that the undergarment (1) forms a waist opening (5) and a pair of leg openings (6a, 6b) with a crotch region (7) extending between the leg openings (6a, 6b), wherein a central longitudinal axis (y) of the undergarment (1) extends along the one or more fabric panels (2) of the undergarment (1) in a direction from the back side (4) and towards the front side (3), and a transversal axis (x) of the undergarment (1) extends along the one or more fabric panels (2), perpendicular to the central longitudinal axis (y), dividing the undergarment (1) into the front side (3) and said back side (4);
the undergarment (1) further comprising an absorbent assembly (10) being arranged in at least part of the crotch region (7) and comprising a wearer facing top layer (15), a moisture barrier (12), and optionally one or more intermediate layers (11, 16) being superimposed along a height axis (z), perpendicular to the longitudinal axis (y) and the transversal axis (x), between the wearer facing top layer (15) and the moisture barrier (12), the wearer facing top layer (15) defining a circumferential outer edge (20) of the absorbent assembly (10), and the undergarment (1) being **characterized in that**
at least a portion of the circumferential outer edge (20) of the absorbent assembly (10) comprises one or more sealed edge sections (21a, 21b) wherein a bottom surface of the wearer facing top layer (15) is sealingly attached to a top surface of the moisture barrier (12), and a bottom surface of the moisture barrier (12) or a bottom surface of the wearer facing top layer (15) is directly attached to a top surface of at least one fabric panel (2), the sealed edge sections (21a, 21b) providing continuous sealing along the sealed edge section (21a, 21b) and between the wearer facing top layer (15) and the moisture barrier (12).

2. The undergarment according to claim 1, wherein at least a portion of the circumferential outer edge (20) forms a front sealed edge section (21a) of the absorbent assembly (10) and faces the front (3) of the undergarment (1).

3. The undergarment according to claim 1, wherein at least a portion of the circumferential outer edge (20) forms a rear sealed edge section (21b) of the absorbent assembly (10) and faces the back (4) of the undergarment (1).

4. The undergarment according to any one of the preceding claims, wherein the absorbent assembly comprises a front sealed edge section (21a) facing the front (3) of the undergarment (1) and a back sealed edge section (21b) facing the back (4) of the undergarment (1).

5. The undergarment according to any one of the preceding claims, wherein, a first fluid seal (24a) is arranged to extend between the bottom surface of the wearer facing top layer (15) and the top surface of the moisture barrier (12) of the one or more sealed edge sections (21a, 21b).

6. The undergarment according to claim 5, wherein the first fluid seal (24a) extends along the circumferential outer edge (20) of the one or more sealed edge sections (21a, 21b) at a distance from the circumferential outer edge (20) of less than 5 mm, such as less than 2 mm, such as less than 1 mm.

7. The undergarment according to any one of claims 5 or 6, wherein a second fluid seal (24b) is arranged to extend between the bottom surface of the moisture barrier (12) and the top surface of the at least one fabric panel (2) along the circumferential outer edge (20) at a distance from the circumferential outer edge (20) in the one or more sealed edge sections (21a, 21b).

8. The undergarment according to claim 7, wherein the distance is less than 5 mm, such as less than 2 mm, such as less than 1 mm.

9. The undergarment according to claim 5, wherein in the one or more sealed edge sections (21a, 21b), the wearer facing top layer (15) extends beyond the moisture barrier (12), and the bottom surface of the wearer facing top layer (15) is directly attached to the top surface of the fabric panel (2) by means of a second fluid seal (24b).

10. The undergarment according to claim 9, wherein the second fluid seal (24b) is arranged to extend between the bottom surface of the wearer facing top layer (15) and the top surface of the at least one fabric panel (2) along the circumferential outer edge (20) at a distance from the circumferential outer edge (20) in the one or more sealed edge sections (21a, 21b).

11. The undergarment according to claim 10, wherein the distance is less than 5 mm, such as less than 2 mm, such as less than 1 mm.

12. The undergarment according to any one of claims 5 and 6, wherein in the one or more sealed edge sections (21a, 21b) the first and second fluid seals (24a,24b) substantially coincide with the circumferential outer edge (20) of the absorbent assembly (10).

13. The undergarment according to any one of claims 5-12, wherein the first and/or second fluid seals (24a,24b) is a fluid sealing joint.

14. The undergarment according to claim 13, wherein the fluid sealing joint is a fluid sealing adhesive joint, a hot melt adhesive joint, or a fluid sealing welded joint, a heat welded joint or an ultrasonic welded joint.

15. The undergarment according to claim 13, wherein the fluid sealing joint is an adhesive tape.

16. The undergarment according to any one of claims 13-15, wherein the wearer facing top layer (15) and the moisture barrier (12) are directly joined by at least one of the fluid sealing joints (24a, 24b).

17. The undergarment according to any one of claims 13-15, wherein the moisture barrier (12) and fabric panel (2) are directly joined by at least one of the fluid sealing joints (24a, 24b).

18. The undergarment according to any one of claims 13-15, wherein the wearer facing top layer (15) and the fabric panel (2) are directly joined by at least one of the fluid sealing joints (24a, 24b).

19. The undergarment according to any one of the preceding claims, wherein a pair of sealed assembly side portions (30a, 30b) are located opposite to each other and on each side of the longitudinal axis (y), in each sealed assembly side portion (30a, 30b) a top surface of the wearer facing top layer (15) is sealingly attached to the moisture barrier (12) or to the one or more fabric panels (2), and wherein optionally each sealed assembly side portion (30a,30b) extends longitudinally along at least a part of the crotch region (7) of the undergarment (1).

20. The undergarment according to claim 19, wherein, in each sealed assembly side portion (30a, 30b), at least a part of the circumferential outer edge (20) of the absorbent assembly (10) follows the contour of a respective leg opening (6a, 6b) of the undergarment (1).

21. The undergarment according to any one of claims 19-20, wherein each sealed assembly side portion (30a, 30b) comprises a fluid sealing member (35a,35b) providing a continuous sealing between the top surface of the wearer facing top layer (15) and a bottom surface of the moisture barrier (12) or a bottom surface of the one or more fabric panels (2) in the sealed absorbent assembly side portion (30a, 30b).

22. The undergarment according to any one of claims 19 to 21, wherein each of the fluid sealing members (35a, 35b) in the sealed assembly side portions (30a, 30b) extends continuously over a longitudinal side sealing length (SSL).

23. The undergarment according to claim 23, wherein the longitudinal side sealing length (SSL) is at least 5 cm, such as at least 10 cm.

24. The undergarment according to claim 23, wherein the longitudinal side sealing length (SSL) is less than 45 cm, such as less than 35 cm.

25. The undergarment according to claim 24, wherein the rear sealed edge section (21b) of the absorbent assembly (10) is longitudinally located between the rearmost longitudinal end of the sealed absorbent assembly side portion (30a, 30b) and the rearmost end of the absorbent assembly (10), and the front sealed edge section (21a) of the absorbent assembly (10) is longitudinally located between the frontmost longitudinal end of the sealed absorbent assembly side portion (30a, 30b) and the frontmost end of the absorbent assembly (10).

26. The undergarment according to any one of the preceding claims when combined with claim 2 and/or 3, wherein the rear sealed edge section (21b) of the absorbent assembly (10) has a Rear Edge Length (REL) along the longitudinal axis (y) and/or the front sealed edge section (21a) of the absorbent assembly (10) has a Front Edge Length (FEL) along the longitudinal axis (y), and wherein the Rear Edge Length (REL) and/or the Front Edge Length (FEL) is at least 2 mm, such as at least 4 mm, or at least 2 cm, such as at least 4 cm or at least 10 cm.

27. A washable and reusable absorbent assembly (10) for an undergarment (1), the assembly (10) comprising a wearer facing top layer (15), a moisture barrier (12), and optionally one or more intermediate layers (11, 16) being superimposed along a height axis (z), perpendicular to the longitudinal axis (y) and the transversal axis (x), between the wearer facing top layer (15) and the moisture barrier (12),
the wearer facing top layer (15) defining a circumferential outer edge (20) of the absorbent assembly (10), and the absorbent assembly (10) being
**characterized in that**
at least a portion of the circumferential outer edge (20) of the absorbent assembly (10) comprises one or more sealed edge sections (21a, 21b) wherein a bottom surface of the wearer facing top layer (15) is sealingly attached to a top surface of the moisture barrier (12), and a bottom surface of the moisture barrier (12) or the wearer facing top layer (15) is configured to be directly attached to a top surface of at least one fabric panel (2) of the undergarment, the sealed edge sections providing continuous sealing along the sealed edge section and between the wearer facing top layer (15) and the moisture barrier (12).

28. The washable and reusable absorbent assembly (10) according to claim 27 and combined with any of the features of claims 2 to 15 and/or 17 to 26.
